# FASCICULE DE BREVET EUROPEEN

(11) **EP 0 889 718 B1**
(45) Date de publication et mention de la délivrance du brevet: **18.09.2002**
(21) Numéro de dépôt: 97916486.0
(22) Date de dépôt: 24.03.1997
(51) Int. Cl.: A61K 7/13

(54) **COMPOSITIONS DE TEINTURE DES FIBRES KERATINIQUES CONTENANT DES PYRROLO-AZOLES; UTILISATION COMME COUPLEURS; PROCEDE DE TEINTURE**
ZUSAMMENSETZUNG ZUR FÄRBUNG VON KERATINISCHEN FASERN ENTHALTEND PYRROLOAZOLE ALS KUPPLER, UND FÄRBEVERFAHREN
KERATIN FIBRE DYE COMPOSITIONS CONTAINING PYRROLO-AZOLE COMPOUNDS, USE THEREOF AS COUPLERS, AND DYEING METHOD

(30) Priorité: 22.03.1996 FR 9603627
(43) Date de publication de la demande: 13.01.1999
(73) Titulaire: L'OREAL, 75008 Paris (FR)
(72) Inventeur: VIDAL, Laurent, F-75013 Paris (FR); MALLE, Gérard, F-77100 Meaux (FR)
(74) Mandataire: Miszputen, Laurent
(86) Numéro de dépôt international: FR9700517
(87) Numéro de publication internationale: WO97035554

(56) Documents cités:
- EP-A- 0 030 680
- EP-A- 0 309 652
- DE-A- 3 731 395
- DE-A- 4 009 097

## Description

L'invention a pour objet une composition pour la teinture des fibres kératiniques en particulier des cheveux humains, contenant au moins un composé pyrrolo-azole comme coupleur et au moins une base d'oxydation.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou para-aminophénols, des composés hétérocycliques, appelés généralement bases d'oxydation. Les précurseurs de colorants d'oxydation, ou base d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est-à-dire permettre d'obtenir des écarts de coloration les plus faibles possible tout au long d'une même fibre kératinique, qui peut être en effet différemment sensibilisée (i.e. abîmée) entre sa pointe et sa racine.

Or, la demanderesse vient maintenant de découvrir qu'il est possible d'obtenir de nouvelles teintures puissantes, peu sélectives et particulièrement résistantes, capables d'engendrer des colorations intenses dans des nuances variées, en utilisant des composés pyrrolo-azoles comme coupleurs en présence d'une base d'oxydation.

Cette découverte est à la base de la présente invention.

L'invention a pour objet une composition pour la teinture des fibres kératiniques et en particulier des fibres kératiniques humaines telles que les cheveux, caractérisée par le fait qu'elle comprend, dans un milieu approprié pour la teinture :
- à titre de coupleur, au moins un composé pyrrolo-azole répondant à l'une des formules (I) et (II) suivantes, ou l'un de ses sels d'addition avec un acide : 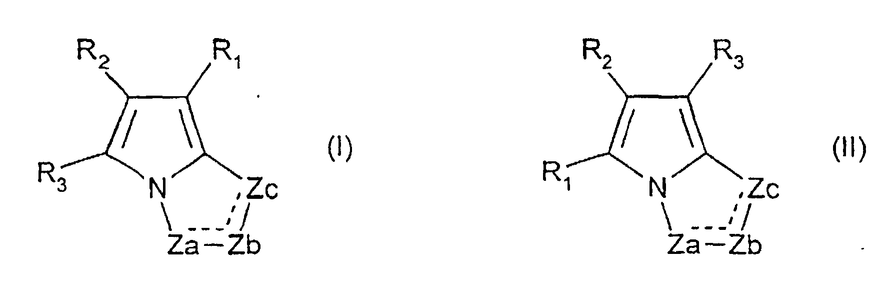 dans lesquelles :
   R₁ représente : un atome d'hydrogène ; un atome d'halogène tel que brome, chlore ou fluor ; un groupe acétylamido ; un radical alcoxy (tel que par exemple : méthoxy; éthoxy, propyloxy, benzyloxy, méthoxyéthoxy, phénoxyéthoxy, 2-cyanoéthoxy, phénéthyloxy, p-chlorobenzyloxy, méthoxyéthylcarbamoylméthoxy); un radical aryloxy (tel que par exemple phénoxy, 4-méthoxyphénoxy, 4-nitrophénoxy, 4-cyanophénoxy, 4-méthanesulfonamidophénoxy, 4-méthanesulfonylphénoxy, 3-méthylphénoxy, 1-naphtyloxy) ; un radical acyloxy (tel que par exemple : acétoxy, propanoyloxy, benzoyloxy, 2,4-dichlorobenzoyloxy, éthoxyalkyloxy, pyruviloyloxy, cinnamoyloxy, myristoyloxy) ; un radical arylthio (tel que par exemple : phénylthio, 4-carboxyphénylthio, 2-éthoxy 5-tert-butylphénylthio, 2-carboxyphénylthio, 4-méthanesulfonylphénylthio) ; un radical alkylthio (tel que par exemple : méthylthio, éthylthio, propylthio, butylthio, 2-cyanoéthylthio, benzylthio, phénéthylthio, 2-(diéthylamino)éthylthio, éthoxyéthylthio, phénoxyéthylthio) ; un radical hétéroarylthio (tel que par exemple : 5-phényl 2,3,4,5-tétrazolylthio, 2-benzothiazolylthio) ; un radical hétéroaryloxy (tel que par exemple : 5-phényl 2,3,4,5-tétrazolyloxy, 2-benzothiazolyloxy); un radical thiocyano ; un radical N,N-diéthyl thiocarbonylthio ; un radical dodécyl-oxythio carbonylthio ; un radical benzènesulfonamido ; un radical N-éthyltoluène sulfonamido ; un radical pentafluorobutanamido ; un radical 2,3,4,5,6-pentafluorobenzamido ; un radical p-cyanophényluréido, un radical N,N-diéthyl-sulfamoylamino; un radical pyrazolyle ; un radical imidazolyle ; un radical triazolyle ; un radical tétrazolyle ; un radical benzimidazolyle ; un radical 1-benzyl 5-éthoxy 3-hydantoïnyle ; un radical 1-benzyl 3-hydantoïnyle ; 5,5-diméthyl 2,4-dioxo 3-oxazolidinyle ; un radical 2-oxy 1,2-dihydro 1-pyridinyle ; un alkylamido ; un arylamido ; un radical NR^{III}R^{IV} avec R^{III} et R^{IV} représentant, identiques ou différents, un alkyle en C₁-C₄ , un hydroxyalkyle ; un carboxyle ; un radical alcoxycarboxylique ;
   R₂ et R₃, indépendamment l'un de l'autre, représentent un atome d'hydrogène ; un atome d'halogène (tel que brome, chlore ou fluor) ; un radical alkyle en C₁-C₅, linéaire ou ramifié, éventuellement substitué par un ou deux radicaux halogène, hydroxy, alcoxy, aryloxy, amino, alkylamino, acyle, acylamino ; un radical alcoxy en C₁-C₄ ; un radical alkylthio en C₁-C₄ ; un radical arylthio ; un radical benzylthio, un radical acyle (tel que acétyle ; 3-phényl propanoyle, benzoyle ; 4-dodécyloxybenzoyle); un radical acylamino ; un radical acyloxy (tel que acétoxy) ; un radical carbamoyle (tel que carbamoyle ; N-éthylcarbamoyle, N-phénylcarbamoyle, N,N-dibutylcarbamoyle) ; N-(2-dodécyl-oxyéthyl) carbamoyle) ; un radical phényle éventuellement substitué par un ou deux groupes halogène, nitro, sulfonyle, alcoxy en C₁-C₄, alkyle en C₁-C₄, trifluoroalkyle en C₁-C₃, amino, alkylamino ; un radical alcoxy carbonyle (tel que méthoxycarbonyle, éthoxycarbonyle, isopropyloxycarbonyle, tertiobutyloxycarbonyle, isobutyloxycarbonyle, butylcarbamoyléthoxy-carbonyle, perfluoro-hexyléthoxy-carbonyle ; un radical aryloxycarbonyle (tel que phénoxycarbonyle, 2,5-amyl phénoxycarbonyle) ; un radical cyano ; un radical nitro ; un radical dialkylphosphono (tel que diméthylphosphono) ; un radical diarylphosphono (tel que diphényl-phosphono) ; un radical dialcoxyphospholyle (tel que diméthoxyphospholyle) ; un radical dialkylphosphinyle (tel que diméthylphosphinyle) ; un radical diarylphosphinyle (tel que diphénylphosphinyle) ; un radical alkyisulfinyle (tel que 3-phénoxy-propyl sulfinyle) ; un radical arylsulfinyle (tel que 3-phénoxypropyl sulfinyle) ; un radical arylsulfonyle (tel que le benzènesulfonyle, toluènesulfonyle) ; un radical alkyl-sulfonyle (méthanesulfonyle, octanesulfonyle) ; un radical sulfonyloxy (tel que méthanesulfonyloxy, toluènesulfonyloxy) ; un radical acylthio (tel que acétylthio, benzoylthio) ; un radical sulfamoyle (tel que N-éthylsulfamoyle, N,N-diisopropylsulfamoyle, N,N-diéthylsulfamoyle) ; un radical thiocyanate ; un radical thiocarbonyle (tel que méthylthiocarbonyle, phénylthio carbonyle) ; Zₐ, Z_{b} et Z_{c}, représentent indépendamment l'un de l'autre, un atome d'azote ou un atome de carbone portant un radical R₄, R₅, R₆ ou R₇ ;
   R₄, R₆ et R₇, identiques ou différents, représentent un atome d'hydrogène ; un radical alkyle en C₁-C₂₀, linéaire ou ramifié, éventuellement substitué par 1 ou 2 radicaux R choisis dans le groupe constitué par halogène, nitro, cyano, hydroxy, alcoxy, aryloxy, amino, alkylamino, acylamino, carbamoyle, sulfonamido, sulfamoyle, imido, alkylthio, arylthio, aryle, alcoxycarbonyle, acyle ; un radical aryle (tel que phényle ou naphtyle), éventuellement substitué par 1 ou 2 radicaux R tels que précédemment définis ; un hétérocycle à 5 ou 6 chaînons possédant au moins un atome d'azote, d'oxygène ou de soufre (tel que pyridyle, quinolyle, pyrrolyle, morpholyle, furanyle, tétrahydrofuranyle, pyrazolyle, triazolyle, tétrazolyle, thiazolyle, oxazolyle, imidazolyle ou thiadiazolyle), éventuellement substitué par 1 ou 2 radicaux R tels que définis précédemment ;
      lorsque R₄, R₆ et R₇ désignent un radical alkyle, un radical aryle ou un l'hétérocycle à 5 ou 6 chaînons (définis ci-dessus), il peut être relié à l'atome de carbone du noyau par l'intermédiaire d'un atome d'oxygène, d'azote ou de soufre (dans ce cas, R₄, R₆ et R₇ deviennent XR₄, XR₆ ou XR₇ avec X = O, NH, S) ;
   R₄, R₆ et R₇ peuvent désigner aussi un atome d'halogène (tel que brome, chlore ou fluor) ; un radical acyle ; un radical sulfonyle ; un radical sulfinyle ; un radical phosphonyle, un radical carbamoyle ; un radical sulfamoyle ; un radical cyano ; un radical siloxy, un radical amino ; un radical acylamino ; un radical acyloxy ; un radical carbamoyloxy ; un radical sulfonamide ; un radical imide ; un radical uréido ; un radical sulfamoylamino ; un radical alcoxycarbonylamino ; un radical aryloxycarbonylamino ; un radical alcoxycarbonyle ; un radical aryloxycarbonyle ; un radical carboxyle ;
   R₅ désigne hydrogène ; halogène ; un radical acyle ; un radical acyloxy ; un radical carbamoyle ; un radical alcoxycarbonyle ; un radical cyano ; un radical nitro ; un radical dialkylphosphono ; un radical diarylphosphono ; un radical dialcoxyphospholyle ; un radical dialkylphosphinyle ; un radical diarylphosphinyle ; un radical alkylsufinyle ; un radical arylsulfinyle ; un radical alkylsulfonyle ; un radical arylsulfonyle ; un radical sulfonyloxy ; un radical acylthio ; un radical sulfamoyle ; un radical thiocyanate ; un radical thiocarbonyle ; un radical aryloxy halogéné (tel que pentafluorophényloxy) ; un alkylamino halogéné (tel que N,N-di(trifluoro méthylamino) ; un alkylthio halogéné (tel que difluorométhylthio) ; un aryle substitué ou non par des groupes électro-attracteurs (par exemple Cl, NO₂, F) ; un hétérocycle (tel que 2-benzoxazolyle, 2-benzothiazolyle ; pyrazolyle, 5-chloro 1-tetrazolyle, 1-pyrrolyle) ;
- et au moins une base d'oxydation.

Parmi les radicaux R₁ des formules (I) et (II), on préfère les radicaux choisis dans le groupe constitué par :
un atome d'hydrogène ; un alcoxy en C₁-C₄ ; phénoxy ; phénoxy substitué par un atome d'halogène, un alkyle en C₁-C₄, un carboxyle, un groupe trifluorométhyle ; un radical acyloxy ; benzyloxy ; alkylthio en C₁-C₄ ; phénylthio ; phénylthio substitué par un atome d'halogène, un alkyle en C₁-C₄, un carboxyle, un groupe trifluorométhyle ; un alkylamido en C₁-C₄ ; phénylamido ; un radical NR^{III}R^{IV} avec R^{III} et R^{IV} représentant, identiques ou différents, un alkyle en C₁-C₄ , un hydroxyalkyle en C₁-C₄ ; un carboxyle ; un radical alcoxycarboxylique en C₁-C₄.

Parmi les radicaux R₁ des formules (I) et (II) définies ci-dessus, on préfère plus particulièrement les radicaux choisis dans le groupe constitué par :
hydrogène ; chlore ou brome ; méthoxy ou éthoxy ; phényloxy ; 4-méthylphényloxy ; acyloxy ; benzyloxy ; méthylthio ou éthylthio ; phénylthio ; 4-méthylphénylthio ; 2-tertio-butylphénylthio ; acétamido ; phénylacétamido ; diméthylamino ; diéthylamino ; éthyl-méthylamino ; (β-hydroxyéthyl)méthylamino.

Et encore plus particulièrement , on préfère les radicaux R₁ choisis dans le groupe constitué par : hydrogène ; chlore ; éthoxy ; phénoxy ; benzyloxy ; acyloxy ; acétamide ; diméthylamino.

Parmi les radicaux R₂ et R₃ des formules (I) et (II), on préfère les radicaux choisis dans le groupe constitué par :
acyle ; acyloxy ; carbamoyle ; alcoxycarbonyle ; aryloxycarbonyle ; cyano ; nitre ; alkylsulfinyle ; arylsulfinyle ; alkylsulfonyle ; arylsulfonyle ; sulfamoyle ; alkyle halogéné ; alkyle en C₁-C₄, hydrogène.

Parmi les radicaux R₂ et R₃ des formules (I) et (II) définies ci-dessus, on préfère plus particulièrement les radicaux choisis dans le groupe constitué par :
acyle (tel que acétyle, éthylcarbonyle, phénylcarbonyle) ; alcoxycarbonyle (tel que méthoxy- ou éthoxycarbonyle) ; nitre ; cyano ; arylsulfonyle (tel que phényisulfonyle) ; carbamoyle (tel que carbamoyle, N-éthylcarbamoyle) ; alkyle halogéné (tel que trifluorométhyle) ; hydrogène ; alkyle en C₁-C₄ (tel que méthyle, éthyle).

Et encore plus particulièrement , on préfère les radicaux R₂ choisis dans le groupe constitué par : cyano ; hydrogène ; méthyle ; phényle et on préfère les radicaux R₃ choisis dans le groupe constitué par : alcoxycarbonyle tel que méthoxycarbonyle, éthoxycarbonyle ; aryloxycarbonyle ; hydrogène ; méthyle ; cyano.

Parmi les radicaux R₄, R₆ et R₇ des formules (I) et (II), on préfère les radicaux choisis dans le groupe constitué par :
un radical alkyle en C₁-C₄, linéaire ou ramifié ; aryle tel que phényle ; phényle substitué par un halogène, un radical méthoxy, un groupe nitro, un groupe cyano, un groupe trifluorométhyle, un groupe amino ; cyano ; nitro ; acylamino ; arylamino ; alkylthio tel que méthylthio, éthylthio ; arylthio tel que phénylthio ; carbamoyle tel que carbamoyle, N-éthylcarbamoyle ; sulfonyle tel que méthylsulfonyle ; alcoxycarbonyle tel que méthoxycarbonyle, éthloxycarbonyle ; aryloxycarbonyle tel que phénoxycarbonyle ; acyle tel que acétyle, éthylcarbonyle ; hydrogène.

Et encore plus particulièrement , on préfère les radicaux R₄, R₆ et R₇ des formules (I) et (II), choisis dans le groupe constitué par : hydrogène ; alkyle en C₁-C₄, linéaire ou ramifié (tel que méthyle, éthyle, isopropyle) ; aryle tel que phényle ; phényle substitué par un halogène, un radical méthoxy, un groupe nitro, un groupe cyano, un groupe trifluorométhyle, un groupe amino.

Parmi les radicaux R₅ des formules (I) et (II) définies ci-dessus, on préfère plus particulièrement les radicaux choisis dans le groupe constitué par :
acyle (tel que acétyle, benzoyle, éthylcarbonyle) ; alcoxycarbonyle (tel que méthoxy- ou éthoxycarbonyle, isopropoxycarbonyle) ; aryloxycarbonyle tel que phénoxycarbonyle ; nitro ; cyano ; arylsulfonyle (tel que phénylsulfonyle) ; alkyle halogéné (tel que trifluorométhyle) ; un hydrogène.

Et encore plus particulièrement , on préfère les radicaux R₅ choisis dans le groupe constitué par : cyano ; alcoxycarbonyle (tel que méthoxy- ou éthoxycarbonyle) ; aryloxycarbonyle tel que phénoxycarbonyle ; alkyle halogéné (tel que trifluorométhyle) ; un hydrogène.

Parmi les composés préférentiels de l'invention répondant à la formule (I), on peut citer ceux choisis dans le groupe constitué par :
(i) les pyrrolo-[1,2-b]-1,2,4-triazoles de formule : 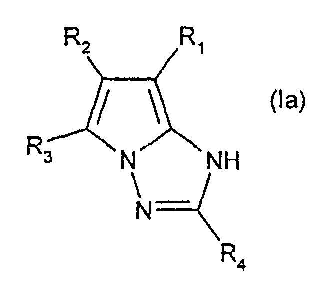
(ii) les pyrrolo-[2,1-c]-1,2,4-triazoles de formule : 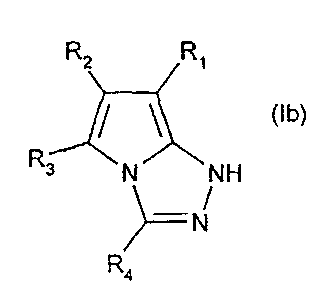
dans lesquelles R₁, R₂, R₃ et R₄ ont les mêmes significations que celles indiquées ci-dessus ;

Comme exemples de composés de formule (la), on peut citer en particulier ceux pour lesquels :
- R₁ désigne hydrogène ou chlore ;
- R₂ et R₃ désignent respectivement : cyano et cyano ; éthyloxycarbonyle et cyano ; trifluorométhyle et cyano ; phénylsulfonyle et cyano ; trifluorométhyle et éthyloxycarbonyle ; éthyloxycarbonyle et éthyloxycarbonyle ; méthyloxycarbonyle et méthyloxycarbonyle ; hydrogène et hydrogène ou hydrogène et méthyle ;
- R₄ désigne méthyle, éthyle, isopropyle, phényle ou hydrogène.

Parmi les composés de formule (la) ci-dessus, on peut tout particulièrement citer :
- le 3,4-dicyano-8-méthyl pyrrolo [1,2-b]-1,2,4-triazole,
- le 3,4-dicyano-8-phényl pyrrolo [1,2-b]-1,2,4-triazole,
- le 3,4-dicyano-8-tertbuyl pyrrolo [1,2-b]-1,2,4-triazole,
- le 5-chloro - 3,4-dicyano-8-méthyl pyrrolo [1,2-b]-1,2,4-triazole,
et leurs sels d'addition avec un acide.

Comme exemples de composés de formule (Ib), on peut citer en particulier ceux pour lesquels :
- R₁ désigne hydrogène ou chlore ;
- R₂ et R₃ désignent simultanément : cyano ou hydrogène ;
- R₄ désigne méthyle, éthyle, isopropyle, phényle ou hydrogène.

Parmi les composés de formule (Ib) ci-dessus, on peut tout particulièrement citer :
- le 5,6-dicyano-3-méthyl- pyrrolo [2,1-c]-1,2,4-triazole,
- le 7-chloro-5,6-dicyano-3-méthyl pyrrolo [2,1-c]-1,2,4-triazole,
et leurs sels d'addition avec un acide.

Parmi les composés préférentiels de l'invention répondant à la formule (II), on peut citer ceux choisis dans le groupe constitué par :
a) les pyrroto-[1,2-b]-1,2,4-triazoles de formule : 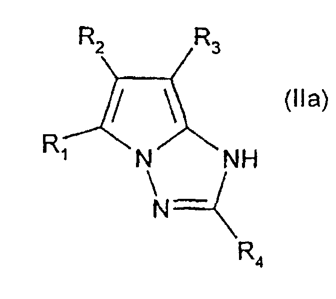
b) les pyrrolo-[2,1-c]-1,2,4-triazoles de formule : 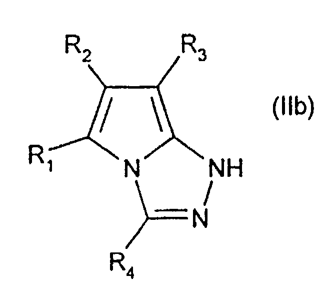
c) les pyrrolo-[1, 2-c]-imidazoles de formule : 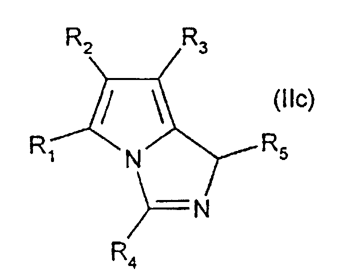
d) les pyrrolo-[1, 2-e]-tétrazoles de formule : 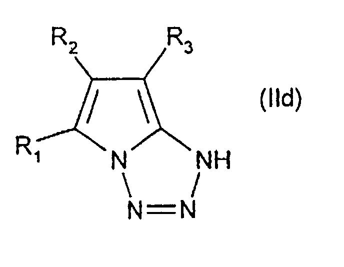
e) les pyrrolo-[1,2-a]-pyrroles de formule : 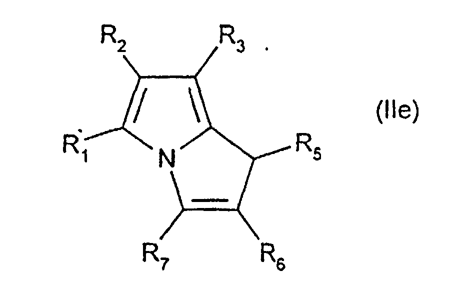
f) les pyrrolo-[1, 2-a]-imidazoles de formule : 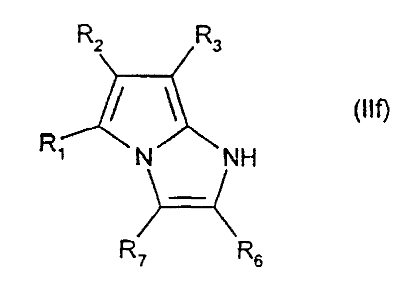
g) les pyrrolo-[1,2-c]-1,2,3-triazoles de formule : 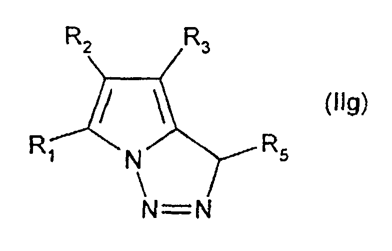
dans lesquelles R₄, R₅, R₆ et R₇ ont les mêmes significations que celles indiquées ci-dessus.

Comme exemples de composés de formule (IIa), on peut citer en particulier ceux pour lesquels :
- R₁ désigne hydrogène ou chlore :
- R₂ et R₃ désignent respectivement : méthoxycarbonyle et cyano ; éthyloxycarbonyle et cyano ; cyano et méthoxycarbonyle ou éthoxycarbonyle ; cyano et trifluorométhyle ; cyano et phénylsulfonyle; méthyloxycarbonyle et méthyloxycarbonyle ; hydrogène et hydrogène ; hydrogène et méthyle ; trifluorométhyle et cyano ou trifluorométhyle et méthyloxycarbonyle ; carboxy et cyano ; cyano et cyano ; éthyloxycarbonyle et éthyloxycarbonyle ; phényle et cyano ; méthyle et hydrogène ;
- R₄ désigne méthyle, éthyle, isopropyle, phényle ou hydrogène.

Parmi les composés de formule (IIa) ci-dessus, on peut tout particulièrement citer :
- le 5-cyano-4-éthoxycarbonyl-8-méthyl pyrrolo [1,2-b]-1,2,4-triazole,
- le 5-cyano-4-carboxy-8-méthyl pyrrolo [1,2-b]-1,2,4-triazole,
- le 4,5-dicyano-8-méthyl pyrrolo [1,2-b]-1,2,4-triazole,
- le 5-cyano-8-méthyl-4-phényl pyrrolo [1,2-b]-1,2,4-triazole,
- le 4, 8-diméthyl pyrrolo [1,2-b]-1,2,4-triazole,
- le 4,5-di-(éthyloxycarbonyl)-8-méthyl pyrrolo [1,2-b]-1,2,4-triazole,
- le 3-chloro-5-cyano-4-éthoxycarbonyl-8-méthyl pyrrolo [1,2-b]-1,2,4-triazole,
- le 5-cyano-4-éthoxycarbonyl-8-phényl pyrrolo [1,2-b]-1,2,4-triazole,
- le 5-cyano-4-carboxy-8-phényl pyrrolo [1,2-b]-1,2,4-triazole,
- le 4,5-dicyano-8-phényl pyrrolo [1,2-b]-1,2,4-triazole,
- le 4,5-di-(éthyloxycarbonyl)-8-phényl pyrrolo [1,2-b]-1,2,4-triazole,
- le 3-chloro-5-cyano-4-éthoxycarbonyl-8-phényl pyrrolo [1,2-b]-1,2,4-triazole,
- le 4-cyano-5-carboxy-8-(2-nitro-5-hydroxyphényl) pyrrolo [1,2-b]-1,2,4-triazole, et leurs sels d'addition avec un acide.

Comme exemples de composés de formule (IIb), on peut citer en particulier ceux pour lesquels :
- R₁ désigne hydrogène ou chlore ;
- R₂ et R₃ désignent respectivement : cyano et méthoxycarbonyle ; méthoxycarbonyle et cyano ; méthoxycarbonyle et méthoxycarbonyle ; hydrogène et hydrogène ou hydrogène et méthyle ; cyano et cyano ; éthyloxycarbonyle et éthyloxycarbonyle ; phényle et cyano ; ter-butyle et cyano ;
- R₄ désigne méthyle, éthyle, isopropyle, phényle ou hydrogène.

Parmi les composés de formule (IIb) ci-dessus, on peut tout particulièrement citer :
- le 6,7-dicyano-3-méthyl pyrrolo [2,1-c]-1,2,4-triazole,
- le 5-chloro-6,7-dicyano-3-méthyl pyrrolo [2,1-c]-1,2,4-triazole,
- le 6,7-di (éthyloxycarbonyl)-3-méthyl pyrrolo [2,1-c]-1,2,4-triazole,
- le 7-cyano-3-méthyl-6-phényl pyrrolo [2,1-c]-1,2,4-triazole,
- le 7-cyano-3-méthyl-6-tertbutyl pyrrolo [2,1-c]-1,2,4-triazole,
et leurs sels d'addition avec un acide.

Comme exemples de composés de formule (IIc), on peut citer en particulier ceux pour lesquels :
- R₁ désigne acétamido, chlore ou hydrogène ;
- R₂ et R₃ désignent respectivement : méthoxycarbonyle et cyano ; cyano et cyano ;
- R₄ désigne hydrogène ;
- R₅ désigne cyano.

Parmi les composés de formule (IIc) ci-dessus, on peut tout particulièrement citer :
- le 6,8-dicyano-5-éthoxycarbonyl pyrrolo [1,2-c] imidazole,
- le 4-chloro-6,8-dicyano-5-éthoxycarbonyl pyrrolo [1,2-c] imidazole,
et leurs sels d'addition avec un acide.

Comme exemples de composés de formule (IId), on peut citer en particulier ceux pour lesquels :
- R₁ désigne hydrogène ou chlore ;
- R₂ et R₃ désignent respectivement : cyano et méthoxycarbonyle ; cyano et cyano ; méthoxyoarbonyle et cyano ; méthoxycarbonyle et méthoxycarbonyle ; hydrogène et hydrogène ou hydrogène et méthyle.

Parmi les composés de formule (IId) ci-dessus, on peut tout particulièrement citer :
- le 6,7-dicyano pyrrolo [1,2-e] tétrazole,
- le 6-cyano-7-éthoxycarbonyl pyrrolo [1,2-e] tétrazole,
- le 5-chloro-6,7-dicyano pyrrolo [1,2-e]tétrazole,
et leurs sels d'addition avec un acide.

Comme exemples de composés de formule (IIe), on peut citer en particulier ceux pour lesquels:
- R₁ désigne hydrogène ou chlore ;
- R₂ et R₃ désignent respectivement : cyano et méthoxycarbonyle ;
- R₅ désigne trifluorométhyle ;
- R₆ désigne phényle ou méthyle ;
- R₇ désigne méthyle.

Comme exemples de composés de formule (IIf), on peut citer en particulier ceux pour lesquels :
- R₁ désigne hydrogène ou chlore ;
- R₂, R₃, R₆ et R₇ désignent respectivement :
   méthoxycarbonyle / cyano /cyano / phényle ;
   cyano / méthoxycarbonyle / cyano / phényle ;
   cyano / méthoxycarbonyle / méthoxycarbonyle / phényle ;
   hydrogène / hydrogène / hydrogène / hydrogène ;
   hydrogène / hydrogène / méthyle / méthyle.

Parmi les composés de formule (IIf) ci-dessus, on peut tout particulièrement citer :
- le 2,3,7-tricyano-6méthyl pyrrolo [1,2-a]- imidazole,
- le 2,3,7-tricyano-6-trifluorométhyl pyrrolo [1,2-a]- imidazole,
- le 2,3,7-tricyano-6-tertbutyl pyrrolo [1,2-a]- imidazole,
- le 2,3,7-tricyano-6-phényl pyrrolo [1,2-a]- imidazole,
- le 2,3,7-tricyano-6-éthoxycarbonyl pyrrolo [1,2-a]- imidazole,
- le 5-chloro-2,3,7-tricyano-6-tertbutyl pyrrolo [1,2-a]- imidazole,
- le 5-chloro-2,3,7-tricyano-6-phényl pyrrolo [1,2-a]- imidazole,
- le 7-cyano-6-éthoxycarbonyl pyrrolo [1,2-a]- benzimidazole,
- le 7-cyano-6-phényl pyrrolo [1,2-a]- benzimidazole,
- le 7-amido-6-éthoxycarbonyl pyrrolo [1,2-a]- benzimidazole,
et leurs sels d'addition avec un acide.

Comme exemples de composés de formule (llg), on peut citer en particulier ceux pour lesquels :
- R₁ désigne hydrogène ou chlore ;
- R₂ désigne cyano ;
- R₃ désigne méthoxycarbonyle ; éthyloxycarbonyle ;
- R₅ désigne cyano.

Parmi les composés de formule (IIg) ci-dessus, on peut tout particulièrement citer :
- le 5,6,8-tricyano pyrrolo [1,2-c]-1,2,3-triazole,
- le 5,8-dicyano-6-éthoxycarbonyl pyrrolo [1,2-c]-1,2,3-triazole,
- le 4-chloro-5,8-dicyano-6-éthoxycarbonyl pyrrolo [1,2-c]-1,2,3-triazole,
et leurs sels d'addition avec un acide.

Les sels d'addition avec un acide des composés de l'invention peuvent être choisis notamment parmi les chlorhydrates, les bromhydrates, les tartrates, les tosylates, les benzènesulfonates, les sulfates, les lactates et les acétates.

Les composés de la présente invention, leurs intermédiaires de synthèse et leurs procédés de préparation sont décrits dans les brevets et demandes de brevets US 5 256 526, EP-A-557 851, EP-A-578 248, EP-A-518 238, EP-A-456 226, EP-A-488 909, EP-A-488 248, et dans les publications suivantes :
- D.R. Liljegren Ber. 1964, 3436 ;
- E.J. Browne, J.C.S., 1962, 5149 ;
- P. Magnus, J.A.C.S., 1990, 112, 2465 ;
- P. Magnus, J.A.C.S., 1987, 109, 2711 ;
- Angew. Chem. 1960, 72, 956 ;
- et Rec. Trav. Chim. 1961, 80, 1075.

Le ou les composés de formule (I) ou (II) représentent de préférence de 0,0005 à 12 % en poids environ du poids total de la composition tinctoriale et plus particulièrement de 0,005 à 6 % en poids.

La nature de la ou des bases d'oxydation pouvant être utilisées dans la composition tinctoriale selon l'invention n'est pas critique. Cette ou ces bases d'oxydation sont de préférence choisies parmi les paraphénylènediamines, les bis-phénylalkylènediamines, les para-aminophénols, les ortho-aminophénols, les bases hétérocycliques, et leurs sels d'addition avec un acide.

Parmi les paraphénylènediamines utilisables à titre de bases d'oxydation dans la composition tinctoriale selon l'invention, on peut notamment citer les composés répondant à la formule (III) suivante, et leurs sels d'addition avec un acide : 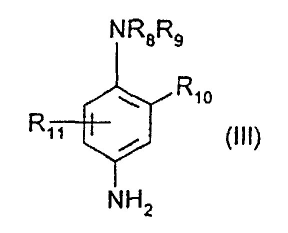 dans laquelle :
R₈ représente un atome d'hydrogène, un radical alkyle en C₁-C₄, monohydroxyalkyle en C₁-C₄, polyhydroxyalkyle en C₂-C₄ ou alcoxy(C₁-C₄) alkyle(C₁-C₄),
R₉ représente un atome d'hydrogène, un radical alkyle en C₁-C₄, monohydroxyalkyle en C₁-C₄ ou polyhydroxyalkyle en C₂-C₄,
R₁₀ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄,
R₁₁ représente un atome d'hydrogène ou un radical alkyle en C₁-C₄.

Dans la formule (III) ci-dessus, et lorsque R₁₀ est différent d'un atome d'hydrogène, alors R₈ et R₉ représentent de préférence un atome d'hydrogène et R₁₀ est de préférence identique à R₁₁, et lorsque R₁₀ représente un atome d'halogène, alors R₈, R₉ et R₁₀ représentent de préférence un atome d'hydrogène.

Parmi les paraphénylènediamines de formule (III) ci-dessus, on peut plus particulièrement citer la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylène-diamine, la N,N-bis-(β-hydroxyéthyl) paraphénylènediamine, le 4-amino 1-(β-méthoxyéthyl)amino benzène, la 2-chloro paraphénylènediamine, et leurs sels d'addition avec un acide.

Parmi les bis-phénylalkylènediamines utilisables à titre de bases d'oxydation dans la composition tinctoriale selon l'invention, on peut notamment citer les composés répondant à la formule (IV) suivante, et leurs sels d'addition avec un acide : 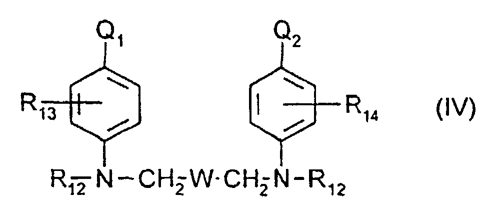 dans laquelle :
Q₁ et Q₂, identiques ou différents, représentent un radical hydroxyle ou NHR₁₅ dans lequel R₁₅ représente un atome d'hydrogène ou un radical alkyle en C₁-C₄, R₁₂ représente un atome d'hydrogène, un radical alkyle en C₁-C₄, monohydroxyalkyle en C₁-C₄, polyhydroxyalkyle en C₂-C₄ ou aminoalkyle en C₁-C₄ dont le reste amino peut être substitué,
R₁₃ et R₁₄, identiques ou différents, représentent un atome d'hydrogène ou d'halogène ou un radical alkyle en C₁-C₄,
W représente un radical pris dans le groupe constitué par les radicaux suivants :

   -(CH₂)ₙ ; -(CH₂)ₘ-O-(CH₂)ₘ ; -(CH₂)ₘ-CHOH-(CH₂)ₘ

   et 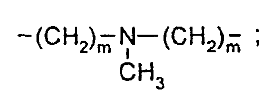
dans lesquels n est un nombre entier compris entre 0 et 8 inclusivement et m est un nombre entier compris entre 0 et 4 inclusivement.

Parmi les bis-phénylalkylènediamines de formules (IV) ci-dessus, on peut plus particulièrement citer le N,N'-bis-(β-hydroxyéthyl) N,N'-bis-(4'-aminophényl) 1,3-diamino 2-propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

Parmi ces bis-phénylalkylènediamines de formule (IV), le N,N'-bis-(β-hydroxyéthyl) N,N'-bis-(4'-aminophényl) 1,3-diamino propanol ou l'un de ses sels d'addition avec un acide sont particulièrement préférés.

Parmi les para-aminophénols utilisables à titre de bases d'oxydation dans la composition tinctoriale selon l'invention, on peut notamment citer les composés répondant à la formule (V) suivante, et leurs sels d'addition avec un acide : 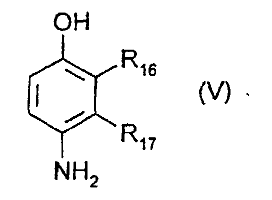 dans laquelle :
R₁₆ représente un atome d'hydrogène, un radical alkyle en C₁-C₄, monohydroxyalkyle en C₁-C₄, alcoxy(C₁-C₄)alkyle(C₁-C₄) ou aminoalkyle en C₁-C₄,
R₁₇ représente un atome d'hydrogène ou de fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₁₆ ou R₁₇ représente un atome d'hydrogène.

Parmi les para-aminophénols de formule (V) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition avec un acide.

Parmi les ortho-aminophénols utilisables à titre de bases d'oxydation dans la composition tinctoriale selon l'invention, on peut notamment citer le 2-amino phénol, le 2-amino 1-hydroxy 5-méthyl benzène, le 2-amino 1-hydroxy 6-méthyl benzène, le 5-acétamido 2-amino phénol, et leurs sels d'addition avec un acide.

Parmi les bases hétérocycliques utilisables à titre de bases d'oxydation dans la composition tinctoriale selon l'invention, on peut notamment citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diaminopyridine, et leurs sels d'addition avec un acide.

Parmi les dérivés pyrimidiniques, on peut plus particulièrement citer les composés décrits par exemple dans les brevets allemand DE 2 359 399 ou japonais JP 88- 571 et JP 91-333 495, comme la 2,4,5,6-tétra-amino-pyrimidine, la 4-hydroxy 2,5,6-triaminopyrimidine, et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969 et WO 94/08970 comme le 4,5-diamino 1-méthyl pyrazole, le 3,4-diamino pyrazole et le 1-(4'-chlorobenzyl)-4,5-diaminopyrazole et leurs sels d'addition avec un acide.

Selon l'invention, la ou les bases d'oxydation représentent de préférence de 0,0005 à 12 % en poids environ du poids total de la composition tinctoriale, et encore plus préférentiellement de 0,005 à 6 % en poids environ de ce poids.

La composition tinctoriale selon l'invention peut également renfermer un ou plusieurs coupleurs additionnels différents des composés de formule (I) et/ou un ou plusieurs colorants directs de façon à faire varier ou enrichir en reflets les nuances obtenues avec les bases d'oxydation.

Les coupleurs additionnels utilisables dans la composition selon l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, et leurs sels d'addition avec un acide.

Ces coupleurs peuvent notamment être choisis parmi le 2-méthyl 5-amino phénol, le 5-N-(β-hydroxyéthyl)amino 2-méthyl phénol, le 3-amino phénol, le 1,3-dihydroxybenzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, et leurs sels d'addition avec un acide.

Lorsqu'ils sont présents ces coupleurs additionnels représentent de préférence de 0,0005 à 5 % en poids environ du poids total de la composition tinctoriale, et encore plus préférentiellement de 0,005 à 3 % en poids environ de ce poids.

Les sels d'addition avec un acide de la ou des bases d'oxydation et/ou des coupleurs additionnels utilisables dans la composition tinctoriale de l'invention sont notamment choisis parmi les chlorhydrates, les bromhydrates, les sulfates et les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (VI) suivante : 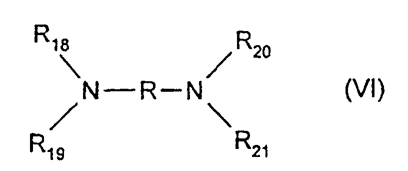 dans laquelle R est un reste propylène éventuellement substitué par un groupement hydroxyle ou un radical alkyle en C₁-C₄ ; R₁₈, R₁₉, R₂₀ et R₂₁, identiques ou différents, représentent un atome d'hydrogène, un radical alkyle en C₁-C₄ ou hydroxyalkyle en C₁-C₄.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet l'utilisation des composés de formule (I) ou (II) ci-dessus, à titre de coupleur, en association avec au moins une base d'oxydation pour la teinture d'oxydation des fibres kératiniques et particulier des fibres kératiniques humaines telles que les cheveux.

Un autre objet de l'invention est un procédé de teinture d'oxydation des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

### EXEMPLES

### EXEMPLES 1 ET 2 DE TEINTURE EN MILIEU ALCALIN

On a préparé les compositions tinctoriales, conformes à l'invention, suivantes (teneurs en grammes) :

| **Exemple** | **1** | **2** |
|---|---|---|
| 5-cyano-4-éthoxycarbonyl-8-méthyl pyrrolo [1,2-b]-1,2,4-triazole (coupleur) | 0,654 | 0,654 |
| 4-amino 1-(β-méthoxyéthyl)amino benzène (base d'oxydation) | 0,498 | - |
| 4,5-diamino 1,3-diméthyl pyrazole (base d'oxydation) | - | 0,384 |
| Support de teinture commun | n°1 | n°1 |
| Eau déminéralisée q.s.p. | 100 g | 100 g |
| NB : le 5-cyano-4-éthoxycarbonyl-8-méthyl pyrrolo [1,2-b]-1,2,4-triazole a été préparé selon le procédé de préparation décrit dans la demande de brevet EP-A-518 238. | | |

### Support de teinture commun n°1 :

- Ethanol 20 g
- Ammoniaque à 20% de NH₃ 10 g
- Métabisulfite de sodium 0,228 g
- Agent séquestrant q.s.

Au moment de l'emploi, chaque composition tinctoriale des exemples 1 et 2 ci-dessus a été mélangée avec un poids égal d'une solution de peroxyde d'hydrogène à 20 volumes (6% en poids).

Chaque mélange obtenu a été appliqué pendant 30 minutes, sur des mèches de cheveux gris naturels à 90 % de blancs, permanentés ou non, à raison de 10 g pour 1 g de cheveux. Après rinçage, lavage avec un shampooing standard et séchage, les mèches ont été teintes dans les nuances figurant dans le tableau 1 ci-dessous :

**TABLEAU 1**

| **Exemple** | **pH du mélange** | **Nuance obtenue sur cheveux gris naturels à 90% de blancs** | **Nuance obtenue sur cheveux gris à 90% de blancs permanentés** |
|---|---|---|---|
| 1 | 9,9 | Vert bouteille | Vert bouteille |
| 2 | 9,9 | Jaune cuivré | Orangé cuivré |

### EXEMPLES 3 à 6 DE TEINTURE EN MILIEU NEUTRE

On a préparé les compositions tinctoriales, conformes à l'invention, suivantes (teneurs en grammes) :

| **Exemple** | **3** | **4** | **5** | **6** |
|---|---|---|---|---|
| 5-cyano-4-éthoxycarbonyl-8-méthyl pyrrolo [1,2-b]-1,2,4-triazole (coupleur) | 0,654 | 0,654 | - | - |
| 5-cyano-4-phényl-8-méthyl pyrrolo [1,2-b]-1,2,4-triazole (coupleur) | - | - | 0,666 | - |
| 7-amido-6-éthoxycarbonyl pyrrolo [1,2-a]- benzimidazole (coupleur) | - | - | - | 0,813 |
| 4-amino 1-(β-méthoxyéthyl)amino benzène (base d'oxydation) | 0,498 | 0,498 | 0,498 | 0,498 |
| Support de teinture commun | n°2 | n°2 | n°2 | n°2 |
| Eau déminéralisée q.s.p. | 100 g | 100 g | 100 g | 100 g |
| **NB** : Le 5-cyano-4-phényl-8-méthyl pyrrolo [1,2-b]-1,2,4-triazole a été préparé selon le procédé décrit dans le brevet US 5 256 526, et le 7-amido-6-éthoxycarbonyl pyrrolo [1,2-a]- benzimidazole a été préparé selon le procédé décrit dans la demande de brevet EP-A-518 238. | | | | |

### Support de teinture commun n°2 :

- Ethanol 20,0 g
- Tampon K₂HPO₄/KH₂PO₄ (1.5 M / 1 M) 10,0 g
- Métabisulfite de sodium 0,228 g
- Agent séquestrant q.s.

Au moment de l'emploi, chacune des compositions tinctoriales des exemples 3 et 6 ci-dessus a été mélangée avec un poids égal d'une solution de peroxyde d'hydrogène à 20 volumes (6% en poids).

Au moment de l'emploi, chacune des compositions tinctoriales des exemples 4 et 5 ci-dessus a été mélangée avec une quantité égale en poids d'une solution aqueuse de persulfate d'ammonium à 6.10⁻³ mole %.

Chaque mélange obtenu a été appliqué pendant 30 minutes, sur des mèches de cheveux gris naturels à 90 % de blancs, permanentés ou non, à raison de 10 g pour 1 g de cheveux. Après rinçage, lavage avec un shampooing standard et séchage, les mèches ont été teintes dans les nuances figurant dans le tableau 2 ci-dessous :

**TABLEAU 2**

| **Exemple** | **pH du mélange** | **Nuance obtenue sur cheveux gris naturels à 90% de blancs** | **Nuance obtenue sur cheveux gris à 90% de blancs permanentés** |
|---|---|---|---|
| **3** | 6,8 | Gris vert-bleu | Gris vert-bleu |
| **4** | 6,8 | Gris bleu-violacé | Gris bleu-violacé |
| **5** | 6,8 | Bleu violacé | Bleu violacé |
| **6** | 6,8 | Gris bleu-vert | Gris bleu-vert |

## Revendications

1. Composition pour la teinture des fibres kératiniques et en particulier des fibres kératiniques humaines telles que les cheveux, **caractérisée par le fait qu'**elle comprend, dans un milieu approprié pour la teinture:
- à titre de coupleur, au moins un composé pyrrolo-azole ou l'un de ses sels d'addition avec un acide répondant à l'une des formules suivantes : 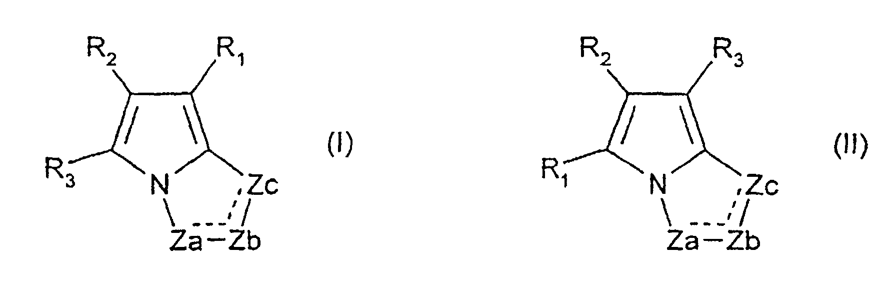 dans lesquelles :
R₁ représente : un atome d'hydrogène ; un atome d'halogène ; un radical acétylamido ; un radical alcoxy ; un radical aryloxy ; un radical acyloxy ; un radical arylthio ; un radical alkylthio ;un radical hétéroarylthio ; un radical hétéroaryloxy ; un radical thiocyano ; un radical N,N-diéthyl thiocarbonylthio ; un radical dodécyloxythio carbonylthio ; un radical benzène-sulfonamido; un radical N-éthyltoluène sulfonamido ; un radical pentafluorobutanamido ; un radical 2,3,4,5,6-pentafluoro-benzamido ; un radical p-cyanophényluréido, un radical N,N-diéthyl-sulfamoylamino ; un radical pyrazolyle ; un radical imidazolyle ; un radical triazolyle ; un radical tétrazolyle ; un radical benzimidazolyle ; un radical 1-benzyl 5-éthoxy 3-hydantoïnyle ; un radical 1-benzyl 3-hydantoïnyle ; 5,5-diméthyl 2,4-dioxo 3-oxazolidinyle ; un radical 2-oxy 1,2-dihydro 1-pyridinyle ; un alkylamido ; un arylamido ; acétylamido ; un radical NR^{III}R^{IV} avec R^{III} et R^{IV} représentant, identiques ou différents, un alkyle en C₁-C₄ , un hydroxyalkyle ; un carboxyle ; un radical alcoxycarboxylique ;
R₂ et R₃, indépendamment l'un de l'autre, représentent un atome d'hydrogène ; un atome d'halogène ; un radical alkyle en C₁-C₅, linéaire ou ramifié, éventuellement substitué par un ou deux radicaux halogène, hydroxy, alcoxy, aryloxy, amino, alkylamino, acyle, acylamino ; un radical alcoxy en C₁-C₄ ; un radical alkylthio en C₁-C₄ ; un radical arylthio ; un radical benzylthio, un radical acyle ; un radical acylamino ; un radical acyloxy ; un radical carbamoyle ; un radical phényle éventuellement substitué par un ou deux groupes halogène, nitro, sulfonyle, alcoxy en C₁-C₄, alkyle en C₁-C₄, trifluoroalkyle en C₁-C₃, amino, alkylamino ; un radical alcoxycarbonyle ; un radical aryloxycarbonyle ; un radical cyano ; un radical nitro ; un radical dialkylphosphono ; un radical diarylphosphono ; un radical dialcoxyphospholyle ; un radical dialkylphosphinyle ; un radical diarylphosphinyle ; un radical alkylsulfinyle ; un radical arylsulfinyle ; un radical arylsulfonyle ; un radical alkylsulfonyle ; un radical sulfonyloxy ; un radical acylthio ; un radical sulfamoyle ; un radical thiocyanate ; un radical thiocarbonyle ;
Zₐ, Z_{b} et Z_{c}, représentent indépendamment l'un de l'autre, un atome d'azote ou un atome de carbone portant un radical R₄, R₅, R₆ ou R₇ ;
R₄, R₆ et R₇, identiques ou différents, représentent un atome d'hydrogène ; un radical alkyle en C₁-C₂₀, linéaire ou ramifié, éventuellement substitué par 1 ou 2 radicaux R choisis dans le groupe constitué par halogène, nitro, cyano, hydroxy, alcoxy, aryloxy, amino, alkylamino, acylamino, carbamoyle, sulfonamido, sulfamoyle, imido, alkylthio, arylthio, aryle, alcoxycarbonyle, acyle ; un radical aryle éventuellement substitué par 1 ou 2 radicaux R tels que précédemment définis ; un hétérocycle à 5 ou 6 chaînons possédant au moins un atome d'azote, d'oxygène ou de soufre éventuellement substitué par 1 ou 2 radicaux R tels que définis précédemment ;
lorsque R₄, R₆ et R₇ désignent un radical alkyle, un radical aryle ou un l'hétérocycle à 5 ou 6 chaînons (définis ci-dessus), il peut être relié à l'atome de carbone du noyau par l'intermédiaire d'un atome d'oxygène, d'azote ou de soufre (dans ce cas, R₄, R₆ et R₇ deviennent XR₄, XR₆ ou XR₇ avec X = O, NH, S) ;
R₄, R₆ et R₇ peuvent désigner aussi un atome d'halogène ; un radical acyle ; un radical sulfonyle ; un radical sulfinyle ; un radical phosphonyle, un radical carbamoyle ; un radical sulfamoyle ; un radical cyano ; un radical siloxy, un radical amino ; un radical acylamino ; un radical acyloxy ; un radical carbamoyloxy ; un radical sulfonamide ; un radical imide ; un radical uréido ; un radical sulfamoylamino ; un radical alcoxycarbonylamino ; un radical aryloxycarbonylamino ; un radical alcoxycarbonyle ; un radical aryloxycarbonyle ; un radical carboxyie ;
R₅ désigne hydrogène ; halogène ; un radical acyle ; un radical acyloxy ; un radical carbamoyle ; un radical alcoxycarbonyle ; un radical cyano ; un radical nitro ; un radical dialkylphosphono ; un radical diarylphosphono ; un radical dialcoxyphospholyle ; un radical dialkylphosphinyle ; un radical diarylphosphinyle ; un radical alkylsufinyle ; un radical arylsulfinyle ; un radical alkylsulfonyle ; un radical arylsulfonyle ; un radical sulfonyloxy ; un radical acylthio ; un radical sulfamoyle ; un radical thiocyanate ; un radical thiocarbonyle ; un radical aryloxy halogéné ; un radical alkylamino halogéné ; un alkylthio halogéné ; un aryle substitué ou non par des groupes électro-attracteurs; un hétérocycle ;
- et au moins une base d'oxydation.

2. Composition selon la revendication 1, **caractérisée par le fait que** les radicaux R₁ des formules (I) et (II) sont choisis dans le groupe constitué par :
un atome d'hydrogène ; un alcoxy en C₁-C₄ ; phénoxy ; phénoxy substitué par un atome d'halogène, un alkyle en C₁-C₄, un carboxyle, un groupe trifluorométhyle ; un radical acyloxy ; benzyloxy ; alkylthio en C₁-C₄ ; phénylthio ; phénylthio substitué par un atome d'halogène, un alkyle en C₁-C₄, un carboxyle, un groupe trifluorométhyle ; un alkylamido en C₁-C₄ ; phénylamido ; un radical NR^{III}R^{IV} avec R^{III} et R^{IV} représentant, identiques ou différents, un alkyle en C₁-C₄ , un hydroxyalkyle en C₁-C₄ ; un carboxyle ; un radical alcoxycarboxylique en C₁-C₄.

3. Composition selon la revendication 2, **caractérisée par le fait que** les radicaux R₁ des formules (I) et (II) sont choisis dans le groupe constitué par :
hydrogène ; chlore ou brome ; méthoxy ou éthoxy ; phényloxy ; 4-méthylphényloxy ; acyloxy ; benzyloxy ; méthylthio ou éthylthio ; phénylthio ; 4-méthylphénylthio ; 2-tertio-butylphénylthio ; acétamido ; phénylacétamido : diméthylamino ; diéthylamino ; éthyl-méthylamino ; (β-hydroxyéthyl)méthylamino.

4. Composition selon la revendication 2 ou 3, **caractérisée par le fait que** les radicaux R₁ des formules (I) et (II) sont choisis dans le groupe constitué par :
hydrogène ; chlore ; éthoxy ; phénoxy ; benzyloxy ; acyloxy ; acétamido ; diméthylamino.

5. Composition selon l'une quelconque des revendications 1 à 4, **caractérisée par le fait que** les radicaux R₂ et R₃ des formules (I) et (II) sont choisis dans le groupe constitué par :
acyle ; acyloxy ; carbamoyle ; alcoxycarbonyle ; aryloxycarbonyte ; cyano ; nitro ; alkylsulfinyle ; arylsulfinyle ; alkylsulfonyle ; arylsulfonyle ; sulfamoyle ; alkyle halogéné ; alkyle en C₁-C₄, hydrogène.

6. Composition selon la revendication 5, **caractérisée par le fait que** les radicaux R₂ et R₃ des formules (I) et (II) sont choisis dans le groupe constitué par:
acyle ; alcoxycarbonyle ; nitro ; cyano ; arylsulfonyle ; carbamoyle ; alkyle halogéné ; hydrogène ; alkyle en C₁-C₄.

7. Composition selon la revendication 5 ou 6, **caractérisée par le fait que** les radicaux R₂ des formules (I) et (II) sont choisis dans le groupe constitué par :
cyano ; hydrogène ; méthyle ; phényle et que les radicaux R₃ des formules (I) et (II) sont choisis dans le groupe constitué par : alcoxycarbonyle tel que méthoxycarbonyle, éthoxycarbonyle ; aryloxycarbonyle ; hydrogène ; méthyle ; cyano.

8. Composition selon l'une quelconque des revendications 1 à 7, **caractérisée par le fait que** les radicaux R₄, R₆ et R₇ des formules (I) et (II) sont choisis dans le groupe constitué par :
un radical alkyle en C₁-C₄, linéaire ou ramifié ; aryle ; phényle substitué par un halogène, un radical méthoxy, un groupe nitro, un groupe cyano, un groupe trifluorométhyle, un groupe amino ; cyano ; nitro ; acylamino ; arylamino ; alkylthio ; arylthio ; carbamoyle ; sulfonyle ; alcoxycarbonyle ; aryloxycarbonyle ; acyle , hydrogène.

9. Composition selon la revendication 8, **caractérisée par le fait que** les radicaux R₄, R₆ et R₇ des formules (I) et (II) sont choisis dans le groupe constitué par :
hydrogène ; alkyle en C₁-C₄, linéaire ou ramifié ; aryle ; phényle substitué par un halogène, un radical méthoxy, un groupe nitro, un groupe cyano, un groupe trifluorométhyle, un groupe amino.

10. Composition selon l'une quelconque des revendications 1 à 9, **caractérisée par le fait que** les radicaux R₅ des formules (I) et (II) sont choisis dans le groupe constitué par :
acyle ; alcoxycarbonyle ; aryloxycarbonyle ; nitro ; cyano ; arylsulfonyle ; alkyle halogéné ; hydrogène.

11. Composition selon l'une quelconque des revendications 1 à 10, **caractérisée par le fait que** les radicaux R₅ sont choisis dans le groupe constitué par : cyano ; alcoxycarbonyle ; aryloxycarbonyle ; alkyle halogéné ; un hydrogène.

12. Composition selon l'une quelconque des revendications 1 à 11, **caractérisée par le fait que** les composés de formule (I) sont choisis dans le groupe constitué par :
(i) les pyrrolo-[1,2-b]-1,2,4-triazoles de formule : 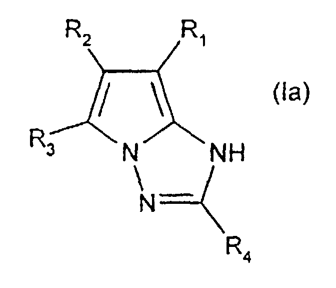
(ii) les pyrrolo-[2,1-c]-1,2,4-triazoles de formule : 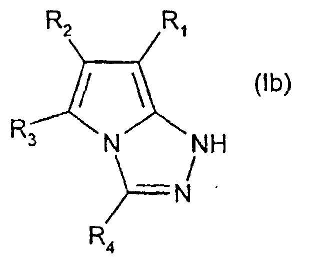
dans lesquelles R₁, R₂, R₃ et R₄ ont les mêmes significations que celles indiquées dans l'une quelconque des revendications 1 à 11.

13. Composition selon la revendication 12, **caractérisée par le fait que** les composés de formule (la), sont ceux pour lesquels :
- R₁ désigne hydrogène ou chlore ;
- R₂ et R₃ désignent respectivement : cyano et cyano ; éthoxycarbonyle et cyano ; trifluorométhyle et cyano ; phénylsulfonyle et cyano ; trifluorométhyle et éthyloxycarbonyle ; éthoxycarbonyle et éthoxycarbonyle ; méthoxycarbonyle et méthoxycarbonyle ; hydrogène et hydrogène ou hydrogène et méthyle ;
- R₄ désigne méthyle, éthyle, isopropyle, phényle ou hydrogène.

14. Composition selon la revendication 12, **caractérisée par le fait que** les composés de formule (Ib), sont ceux pour lesquels :
- R₁ désigne hydrogène ou chlore ;
- R₂ et R₃ désignent simultanément : cyano ou hydrogène ;
- R₄ désigne méthyle, éthyle, isopropyle, phényle ou hydrogène.

15. Composition selon la revendication 12, **caractérisée par le fait que** les composés de formule (I) sont choisis parmi :
- le 3,4-dicyano-8-méthyl pyrrolo [1,2-b]-1,2,4-triazole,
- le 3,4-dicyano-8-phényl pyrrolo [1,2-b]-1,2,4-triazole,
- le 3,4-dicyano-8-tertbuyl pyrrolo [1,2-b]-1,2,4-triazole,
- le 5-chloro-3,4-dicyano-8-méthyl pyrrolo [1,2-b]-1,2,4-triazole,
- le 5,6-dicyano-3-méthyl- pyrrolo [2,1-c]-1,2,4-triazole,
- le 7-chloro-5,6-dicyano-3-méthyl pyrrolo [2,1-c]-1,2,4-triazole,
et leurs sels d'addition avec un acide.

16. Composition selon l'une quelconque des revendications 1 à 11, **caractérisée par le fait que** les composés de formule (II) sont choisis dans le groupe constitué par :
a) les pyrrolo-[1,2-b]-1,2,4-triazoles de formule : 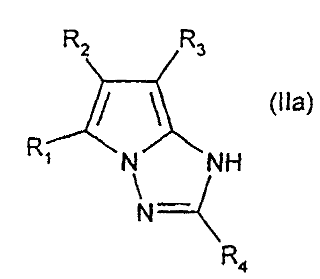
b) les pyrrolo-[2,1-c]-1,2,4-triazoles de formule: 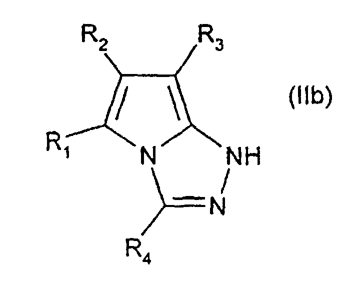
c) les pyrrolo-[1,2-c]-imidazoles de formule : 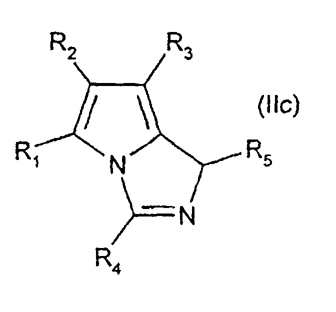
d) les pyrrolo-[1, 2-e]-tétrazoles de formule : 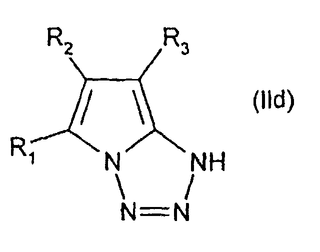
e) les pyrrolo-[1, 2-a]-pyrroles de formule : 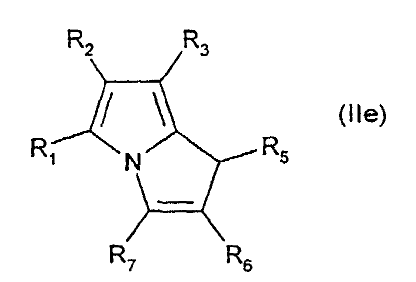
f) les pyrrolo-[1, 2-a]-imidazoles de formule : 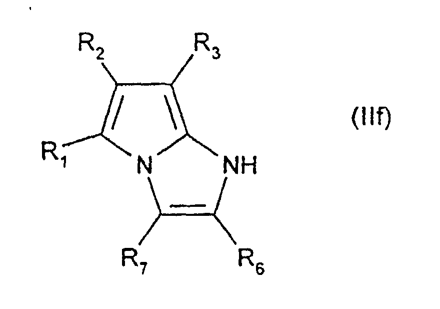
g) les pyrrolo-[1, 2-c]-1,2,3-triazoles de formule : 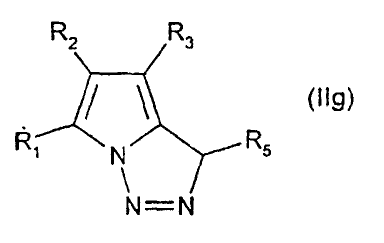
dans lesquelles R₁, R₂, R₃, R₄, R₅, R₆ et R₇ ont les mêmes significations que celles indiquées dans l'une quelconque des revendications 1 à 10.

17. Composition selon la revendication 16, **caractérisée par le fait que** les composés de formule (IIa), sont ceux pour lesquels :
- R₁ désigne hydrogène ou chlore ;
- R₂ et R₃ désignent respectivement : méthoxycarbonyle et cyano ; éthyloxycarbonyle et cyano ; cyano et méthoxycarbonyle ou éthoxycarbonyle ; cyano et trifluorométhyle ; cyano et phénylsulfonyle; méthyloxycarbonyle et méthyloxycarbonyle ; hydrogène et hydrogène ; hydrogène et méthyle ; trifluorométhyle et cyano ou trifluorométhyle et méthyloxycarbonyle ; carboxy et cyano ; cyano et cyano ; éthyloxycarbonyle et éthyloxycarbonyle ; phényle et cyano ; méthyle et hydrogène ;
- R₄ désigne méthyle, éthyle, isopropyle, phényle ou hydrogène.

18. Composition selon la revendication 16, **caractérisée par le fait que** les composés de formule (IIb), sont ceux pour lesquels :
- R₁ désigne hydrogène ou chlore ;
- R₂ et R₃ désignent respectivement : cyano et méthoxycarbonyle ; méthoxycarbonyle et cyano ; méthoxycarbonyle et méthoxycarbonyle ; hydrogène et hydrogène ou hydrogène et méthyle ; cyano et cyano ; éthyloxycarbonyle et éthyloxycarbonyle ; phényle et cyano ; ter-butyle et cyano ;
- R₄ désigne méthyle, éthyle, isopropyle, phényle ou hydrogène.

19. Composition selon la revendication 16, **caractérisée par le fait que** les composés de formule (IIc), sont ceux pour lesquels :
- R₁ désigne acétamido, hydrogène ou chlore ;
- R₂ et R₃ désignent respectivement : méthoxycarbonyle et cyano ; cyano et cyano ;
- R₄ désigne hydrogène ;
- R₅ désigne cyano.

20. Composition selon la revendication 16, **caractérisée par le fait que** les composés de formule (IId), sont ceux pour lesquels :
- R₁ désigne hydrogène ou chlore ;
- R₂ et R₃ désignent respectivement : cyano et méthoxycarbonyle ; cyano et cyano ; méthoxycarbonyle et cyano ; méthoxycarbonyle et méthoxycarbonyle ; hydrogène et hydrogène ; hydrogène et méthyle.

21. Composition selon la revendication 16, **caractérisée par le fait que** les composés de formule (IIe), sont ceux pour lesquels :
- R₁ désigne hydrogène ou chlore ;
- R₂ et R₃ désignent respectivement : cyano et méthoxycarbonyle ;
- R₅ désigne trifluorométhyle ;
- R₆ désigne phényle ou méthyle ;
- R₇ désigne méthyle.

22. Composition selon la revendication 16, **caractérisée par le fait que** les composés de formule (IIf), sont ceux pour lesquels :
- R₁ désigne hydrogène ou chlore ;
- R₂, R₃, R₆ et R₇ désignent respectivement :
méthoxycarbonyle / cyano / cyano / phényle ;
cyano /méthoxycarbonyle / cyano / phényle ;
cyano / méthoxycarbonyle / méthoxycarbonyle/phényle ;
hydrogène / hydrogène / hydrogène / hydrogène ;
hydrogène / hydrogène / méthyle / méthyle.

23. Composition selon la revendication 16, **caractérisée par le fait que** les composés de formule (IIg), sont ceux pour lesquels :
- R₁ désigne hydrogène ou chlore ;
- R₂ désigne cyano ;
- R₃ désigne méthoxycarbonyle ; éthyloxycarbonyle ;
- R₅ désigne cyano.

24. Composition selon la revendication 16, **caractérisée par le fait que** les composés de formule (II) sont choisis parmi :
- le 5-cyano-4-éthoxycarbonyl-8-méthyl pyrrolo [1,2-b]-1,2,4-triazole,
- le 5-cyano-4-carboxy-8-méthyl pyrrolo [1,2-b]-1,2,4-triazole,
- le 4,5-dicyano-8-méthyl pyrrolo [1,2-b]-1,2,4-triazole,
- le 5-cyano-8-méthyl-4-phényl pyrrolo [1,2-b]-1,2,4-triazole,
- le 4, 8-diméthyl pyrrolo [1,2-b]-1,2,4-triazole,
- le 4,5-di-(éthyloxycarbonyl)-8-méthyl pyrrolo [1,2-b]-1,2,4-triazole,
- le 3-chloro-5-cyano-4-éthoxycarbonyl-8-méthyl pyrrolo [1,2-b]-1,2,4-triazole,
- le 5-cyano-4-éthoxycarbonyl-8-phényl pyrrolo [1,2-b]-1,2,4-triazole,
- le 5-cyano-4-carboxy-8-phényl pyrrolo [1,2-b]-1,2,4-triazole,
- le 4,5-dicyano-8-phényl pyrrolo [1,2-b]-1,2,4-triazole,
- le 4,5-di-(éthyloxycarbonyl)-8-phényl pyrrolo [1,2-b]-1,2,4-triazole,
- le 3-chloro-5-cyano-4-éthoxycarbonyl-8-phényl pyrrolo [1,2-b]-1,2,4-triazole,
- le 4-cyano-5-carboxy-8-(2-nitro-5-hydroxyphényl) pyrrolo [1,2-b]-1,2,4-triazole,
- le 6,7-dicyano-3-méthyl pyrrolo [2,1-c]-1,2,4-triazole,
- le 5-chloro-6,7-dicyano-3-méthyl pyrrolo [2,1-c]-1,2,4-triazole,
- le 6,7-di (éthyloxycarbonyl)-3-méthyl pyrrolo [2,1-c]-1,2,4-triazole,
- le 7-cyano-3-méthyl-6-phényl pyrrolo [2,1-c]-1,2,4-triazole,
- le 7-cyano-3-méthyl-6-tertbutyl pyrrolo [2,1-c]-1,2,4-triazole,
- le 6,8-dicyano-5-éthoxycarbonyl pyrrolo [1,2-c] imidazole,
- le 4-chloro-6,8-dicyano-5-éthoxycarbonyl pyrrolo [1,2-c] imidazole,
- le 6,7-dicyano pyrrolo [1,2-e] tétrazole,
- le 6-cyano-7-éthoxycarbonyl pyrrolo [1,2-e] tétrazole,
- le 5-chloro-6,7-dicyano pyrrolo [1,2-e] tétrazole,
- le 2,3,7-tricyano-6méthyl pyrroto [1,2-a]- imidazole,
- le 2,3,7-tricyano-6-trifluorométhyl pyrrolo [1,2-a]- imidazole,
- le 2,3,7-tricyano-6-tertbutyl pyrrolo [1,2-a]- imidazole,
- le 2,3,7-tricyano-6-phényl pyrrolo [1,2-a]- imidazole,
- le 2,3,7-tricyano-6-éthoxycarbonyl pyrrolo [1,2-a]- imidazole,
- le 5-chloro-2,3,7-tricyano-6-tertbutyl pyrrolo [1,2-a]- imidazole,
- le 5-chloro-2,3,7-tricyano-6-phényl pyrrolo [1,2-a]- imidazole,
- le 7-cyano-6-éthoxycarbonyl pyrrolo [1,2-a]- benzimidazole,
- le 7-cyano-6-phényl pyrroio [1,2-a]- benzimidazole,
- le 5,6,8-tricyano pyrrolo [1,2-c]-1,2,3-triazole,
- le 7-amido-6-éthoxycarbonyl pyrrolo [1,2-a]- benzimidazole,
- le 5,8-dicyano-6-éthoxycarbonyl pyrrolo [1,2-c]-1,2,3-triazole,
- le 4-chloro-5,8-dicyano-6-éthoxycarbonyl pyrrolo [1,2-c]-1,2,3-triazole,
et leurs sels d'addition avec un acide.

25. Composition selon l'une quelconque des revendications précédentes, **caractérisée par le fait que** les sels d'addition avec un acide des composés de formule (I) ou (II), sont choisis parmi les chlorhydrates, les bromhydrates, les tartrates, les tosylates, les benzènesulfonates, les sulfates, les lactates et les acétates.

26. Composition selon l'une quelconque des revendications précédentes, **caractérisée par le fait que** le ou les composés de formule (I) ou (II) représentent de 0,0005 à 12 % en poids du poids total de la composition tinctoriale.

27. Composition selon la revendication 26, **caractérisée par le fait que** le ou les composés de formule (I) ou (II) représentent de 0,005 à 6 % en poids du poids total de la composition tinctoriale.

28. Composition selon l'une quelconque des revendications précédentes, **caractérisée par le fait que** la ou les bases d'oxydation sont choisies parmi les paraphénylènediamines, les bis-phénylalkylènediamines, les paraaminophénols, les ortho-aminophénols, les bases hétérocycliques, et leurs sels d'addition avec un acide.

29. Composition selon l'une quelconque des revendications précédentes, **caractérisée par le fait que** la ou les bases d'oxydation représentent de 0,0005 à 12 % en poids environ du poids total de la composition tinctoriale.

30. Composition selon la revendication 29, **caractérisée par le fait que** la ou les bases d'oxydation représentent de 0,005 à 6 % en poids environ du poids total de la composition tinctoriale.

31. Composition selon l'une quelconque des revendications précédentes, **caractérisée par le fait qu'**elle renferme en outre un ou plusieurs coupleurs additionnels différents des composés de formule (I) ou (II) et/ou un ou plusieurs colorants directs.

32. Composition selon l'une quelconque des revendications précédentes, **caractérisée par le fait que** le milieu approprié pour la teinture (ou support) est constitué par de l'eau ou par un mélange d'eau et d'au moins un solvant organique choisi parmi les alcanols inférieurs en C₁-C₄, le glycérol, les glycols et éthers de glycols, les alcools aromatiques, les produits analogues et leurs mélanges.

33. Composition selon l'une quelconque des revendications précédentes, **caractérisée par le fait qu'**elle présente un pH compris entre 3 et 12.

34. Composition selon l'une quelconque des revendications précédentes, **caractérisée par le fait qu'**elle se présente sous forme de liquides, de crèmes, de gels, ou sous toute autre forme appropriée pour réaliser une teinture des fibres kératiniques, et notamment des cheveux humains.

35. Utilisation des composés de formule (I) ou (II) ou de leurs sels d'addition avec un acide tels que définis dans l'une quelconque des revendications 1 à 25, à titre de coupleurs dans des compositions pour la teinture d'oxydation des fibres kératiniques et en particulier des fibres kératiniques humaines telles que les cheveux, en association avec au moins une base d'oxydation.

36. Procédé de teinture d'oxydation des fibres kératiniques et en particulier des fibres kératiniques humaines telles que les cheveux, **caractérisé par le fait qu'**on applique sur ces fibres au moins une composition tinctoriale telle que définie à l'une quelconque des revendications 1 à 34, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

37. Procédé selon la revendication 36, **caractérisé par le fait que** l'agent oxydant est choisi parmi le peroxyde d'hydrogène, le peroxyde d'urée, les bromates de métaux alcalins, les persels tels que les perborates et persulfates.

38. Dispositif à plusieurs compartiments, ou "kit" de teinture à plusieurs compartiments, dont un premier compartiment renferme une composition tinctoriale telle que définie à l'une quelconque des revendications 1 à 34 et un second compartiment renferme une composition oxydante.

## Patentansprüche

1. Zusammensetzung zum Färben von Keratinfasern und insbesondere menschlichen Keratinfasern, wie dem Haar, **dadurch gekennzeichnet, daß** sie in einem zum Färben geeigneten Medium enthält:
- als Kuppler mindestens ein Pyrrolo-azol der folgenden Formeln oder ein Additionssalz dieser Verbindungen mit einer Säure: 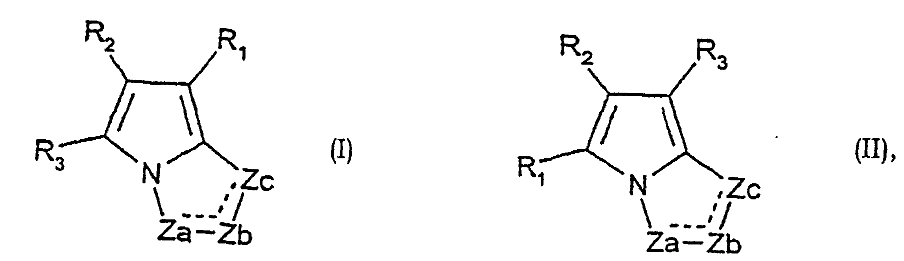 worin bedeuten :
- R₁ Wasserstoff; ein Halogenatom; Acetylamido; Alkoxy; Aryloxy; Acyloxy; Arylthio; Alkylthio; Heteroarylthio; Heteroaryloxy; Thiocyano; N,N-Diethylthiocarbonylthio; Dodecyloxythiocarbonylthio; Benzolsulfonamido; N-Ethyltoluolsulfonamido; Pentafluorbutanamido; 2,3,4,5,6-Pentafluorbenzamido; p-Cyanophenylureido; N,N-Diethylsulfamoylamino; Pyrazolyl; Imidazolyl; Triazolyl; Tetrazolyl; Benzimidazolyl; 1-Benzyl-5-ethoxy-3-hydantoinyl; 1-Benzyl-3-hydantoinyl; 5,5-Dimethyl-2,4-dioxo-3-oxazolidinyl; 2-Oxy-1,2-dihydro-1-pyridinyl; Alkylamido; Arylamido; Acetylamido; NR^{III}R^{IV}, worin R^{III} und R^{IV}, die identisch oder voneinander verschieden sind, C₁₋₄-Alkyl oder Hydroxyalkyl bedeuten; Carboxy, Alkoxycarboxy;
- die Gruppen R₂ und R₃ unabhängig voneinander Wasserstoff; ein Halogenatom; eine geradkettige oder verzweigte C₁₋₅-Alkylgruppe, die gegebenenfalls mit einer oder zwei Gruppen Halogen, Hydroxy, Alkoxy, Aryloxy, Amino, Alkylamino, Acyl oder Acylamino substituiert ist; C₁₋₄-Alkoxy; C₁₋₄-Alkylthio; Arylthio; Benzylthio; Acyl; Acylamino; Acyloxy; Carbamoyl; eine Phenylgruppe, die gegebenenfalls mit einer oder zwei Halogen-, Nitro-, Sulfonyl-, C₁₋₄-Alkoxy-, C₁₋₄-Alkyl-, C₁₋₃-Trifluoralkyl-, Amino- oder Alkylaminogruppen substituiert ist; Alkoxycarbonyl; Aryloxycarbonyl; Cyano; Nitro; Dialkylphosphono; Diarylphosphono; Dialkoxyphospholyl; Dialkylphosphinyl; Diarylphosphinyl; Alkylsulfinyl; Arylsulfinyl; Arylsulfonyl; Alkylsulfonyl; Sulfonyloxy; Acylthio; Sulfamoyl; Thiocyanat; Thiocarbonyl;
- Zₐ, Z_{b} und Z_{c} unabhängig voneinander ein Stickstoffatom oder ein Kohlenstoffatom, das eine Gruppe R₄, R₅, R₆ oder R₇ trägt;
- R₄, R₆ und R₇, die identisch oder voneinander verschieden sind, Wasserstoff; eine geradkettige oder verzweigte C₁₋₂₀-Alkylgruppe, die gegebenenfalls mit einer oder zwei Gruppen R substituiert ist, die ausgewählt sind unter Halogen, Nitro, Cyano, Hydroxy, Alkoxy, Aryloxy, Amino, Alkylamino, Acylamino, Carbamoyl, Sulfonamido, Sulfamoyl, Imido, Alkylthio, Arylthio, Aryl, Alkoxycarbonyl und Acyl; eine Arylgruppe, die gegebenenfalls mit einer oder zwei der oben definierten Gruppen R substituiert ist; einen Heterocyclus mit 5 oder 6 Gliedern, der mindestens ein Stickstoffatom, ein Sauerstoffatom oder ein Schwefelatom aufweist und gegebenenfalls mit einer oder zwei der oben definierten Gruppen R substituiert ist; wenn die Gruppen R₄, R₆ und R₇ eine Alkylgruppe, eine Arylgruppe oder einen Heterocyclus mit 5 oder 6 Gliedern (die oben definiert sind) bedeuten, können sie über ein Sauerstoffatom, ein Stickstoffatom oder ein Schwefelatom an das Kohlenstoffatom des Rings gebunden sein (R₄, R₆ und R₇ wird in diesem Fall zu XR₄, XR₆ und XR₇ mit X = O, NH, S); und
R₄, R₆ und R₇ können auch bedeuten: ein Halogenatom; Acyl; Sulfonyl; Sulfinyl; Phosphonyl; Carbamoyl; Sulfamoyl; Cyano; Siloxy; Amino; Acylamino; Acyloxy; Carbamoyloxy; Sulfonamid; Imid; Ureido; Sulfamoylamino; Alkoxycarbonylamino; Aryloxycarbonylamino; Alkoxycarbonyl; Aryloxycarbonyl; Carboxy;
- die Gruppe R₅ Wasserstoff; Halogen; Acyl; Acyloxy; Carbamoyl; Alkoxycarbonyl; Cyano; Nitro; Dialkylphosphono; Diarylphosphono; Dialkoxyphospholyl; Dialkylphosphinyl; Diarylphosphinyl; Alkylsulfinyl; Arylsulfinyl; Alkylsulfonyl; Arylsulfonyl; Sulfonyloxy; Acylthio; Sulfamoyl; Thiocyanat; Thiocarbonyl; eine halogenierte Aryloxygruppe; eine halogenierte Alkylaminogruppe; eine halogenierte Alkylthiogruppe; eine Arylgruppe, die gegebenenfalls mit elektronenziehenden Gruppen substituiert ist; einen Heterocyclus;
und
- mindestens eine Oxidationsbase.

2. Zusammensetzung nach Anspruch 1, **dadurch gekennzeichnet, daß** die Gruppen R₁ der Formeln (I) und (II) ausgewählt sind unter: Wasserstoff; C₁₋₄- Alkoxy; Phenoxy; Phenoxygruppen, die mit einem Halogenatom, C₁₋₄-Alkyl, Carboxy oder Trifluormethyl substituiert sind; Acyloxy; Benzyloxy; C₁₋₄-Alkylthio; Phenylthio; Phenylthiogruppen, die mit einem Halogenatom, C₁₋₄-Alkyl, Carboxy oder Trifluormethylsubstituiert sind; C₁₋₄-Alkylamido; Phenylamido; NR^{III}R^{IV}, worin R^{III} und R^{IV}, die identisch oder voneinander verschieden sind, C₁₋₄-Alkyl oder C₁₋₄-Hydroxyalkyl bedeuten; Carboxy; oder C₁₋₄-Alkoxycarboxy.

3. Zusammensetzung nach Anspruch 2, **dadurch gekennzeichnet, daß** die Gruppen R₁ der Formeln (I) und (II) unter Wasserstoff; Chlor oder Brom; Methoxy oder Ethoxy; Phenoxy; 4-Methylphenyloxy; Acyloxy; Benzyloxy; Methylthio oder Ethylthio; Phenylthio; 4-Methylphenylthio; 2-t-Butylphenylthio; Acetamido; Phenylacetamido; Dimethylamino; Diethylamino; Ethylmethylamino; und (β-Hydroxyethyl)methylamino ausgewählt sind.

4. Zusammensetzung nach Anspruch 2 oder 3, **dadurch gekennzeichnet, daß** die Gruppen R₁ der Formeln (I) und (II) ausgewählt sind unter: Wasserstoff; Chlor; Ethoxy; Phenoxy; Benzyloxy; Acyloxy; Acetamido; und Dimethylamino.

5. Zusammensetzung nach einem der Ansprüche 1 bis 4, **dadurch gekennzeichnet, daß** die Gruppen R₂ und R₃ der Formeln (I) und (II) ausgewählt sind unter:
Acyl; Acyloxy; Carbamoyl; Alkoxycarbonyl; Aryloxycarbonyl; Cyano; Nitro; Alkylsulfinyl; Arylsulfinyl; Alkylsulfonyl; Arylsulfonyl; Sulfamoyl; halogenierte Alkylgruppen; C₁₋₄-Alkyl; und Wasserstoff.

6. Zusammensetzung nach Anspruch 5, **dadurch gekennzeichnet, daß** die Gruppen R₂ und R₃ der Formeln (I) und (II) ausgewählt sind unter:
Acyl; Alkoxycarbonyl; Nitro; Cyano; Arylsulfonyl; Carbamoyl; halogenierten Alkylgruppen; Wasserstoff; und C₁₋₄-Alkyl.

7. Zusammensetzung nach Anspruch 5 oder 6, **dadurch gekennzeichnet, daß** die Gruppen R₂ der Formeln (I) und (II) ausgewählt sind unter: Cyano; Wasserstoff; Methyl; und Phenyl; und die Gruppen R₃ Formeln (I) und (II) ausgewählt sind unter: Alkoxycarbonyl wie Methoxycarbonyl und Ethoxycarbonyl; Aryloxycarbonyl; Wasserstoff; Methyl; und Cyano.

8. Zusammensetzung nach einem der Ansprüche 1 bis 7, **dadurch gekennzeichnet, daß** die Gruppen R₄, R₆ und R₇ der Formeln (I) und (II) ausgewählt sind unter:
geradkettigen oder verzweigten C₁₋₄-Alkylgruppen; Aryl; Phenylgruppen, die mit einem Halogenatom, Methoxy, Nitro, Cyano, Trifluormethyl oder Amino substituiert sind; Cyano; Nitro; Acylamino; Arylamino; Alkylthio; Arylthio; Carbamoyl; Sulfonyl; Alkoxycarbonyl; Aryloxycarbonyl; Acyl; und Wasserstoff.

9. Zusammensetzung nach Anspruch 8, **dadurch gekennzeichnet, daß** die Gruppen R₄, R₆ und R₇ der Formeln (I) und (II) ausgewählt sind unter: Wasserstoff; geradkettigen oder verzweigten C₁₋₄-Alkylgruppen; Aryl; und Phenylgruppen, die mit einem Halogenatom, Methoxy, Nitro, Cyano, Trifluormethyl oder Amino substituiert sind.

10. Zusammensetzung nach einem der Ansprüche 1 bis 9, **dadurch gekennzeichnet, daß** die Gruppen Rs der Formeln (I) und (II) ausgewählt sind unter: Acyl; Alkoxycarbonyl; Aryloxycarbonyl; Nitro; Cyano; Arylsulfonyl; halogenierten Alkylgruppen; und Wasserstoff.

11. Zusammensetzung nach einem der Ansprüche 1 bis 10, **dadurch gekennzeichnet, daß** die Gruppen R₅ ausgewählt sind unter: Cyano; Alkoxycarbonyl; Aryloxycarbonyl; halogenierten Alkylgruppen; und Wasserstoff.

12. Zusammensetzung nach einem der Ansprüche 1 bis 11, **dadurch gekennzeichnet, daß** die Verbindungen der Formen (I) ausgewählt sind unter:
(i) Pyrrolo-[1,2-b]-1,2,4-triazolen der Formel: 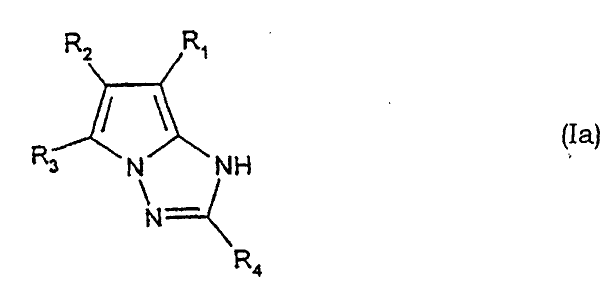
(ii) Pyrrolo-[2,1-c]-1,2,4-triazolen der Formel: 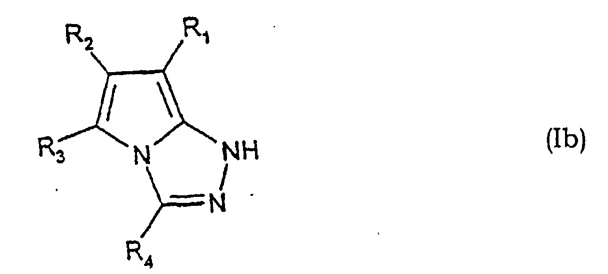
wobei die Gruppen R₁, R₂, R₃ und R₄ die in den Ansprüchen 1 bis 11 angegebenen Bedeutungen aufweisen.

13. Zusammensetzung nach Anspruch 12, **dadurch gekennzeichnet, daß** die Verbindungen der Formel (Ia) Verbindungen sind, worin bedeuten:
- R₁ Wasserstoff oder Chlor;
- R₂ bzw. R₃: Cyano und Cyano; Ethyloxycarbonyl und Cyano; Trifluormethyl und Cyano; Phenylsulfonyl und Cyano; Trifluormethyl und Ethyloxycarbonyl; Ethoxycarbonyl und Ethoxycarbonyl; Methoxycarbonyl und Methoxycarbonyl; Wasserstoff und Wasserstoff oder Wasserstoff und Methyl;
- R₄ Methyl, Ethyl, Isopropyl, Phenyl oder Wasserstoff.

14. Zusammensetzung nach Anspruch 12, **dadurch gekennzeichnet, daß** die Verbindungen der Formel (Ib) Verbindungen sind, worin bedeuten:
- R₁ Wasserstoff oder Chlor;
- R₂ und R₃ gleichzeitig: Cyano oder Wasserstoff;
- R₄ Methyl, Ethyl, Isopropyl, Phenyl oder Wasserstoff.

15. Zusammensetzung nach Anspruch 12, **dadurch gekennzeichnet, daß** die Verbindungen der Formel (I) ausgewählt sind unter:
- 3,4-Dicyano-8-methyl-pyrrolo-[1,2-b]-1,2,4-triazol,
- 3,4-Dicyano-8-phenyl-pyrrolo-[1,2-b]-1,2,4-triazol,
- 3,4-Dicyano-8-t-butyl-pyrrolo-[1,2-b]-1,2,4-triazol,
- 5-Chlor-3,4-dicyano-8-methyl-pyrrolo-[1,2-b]-1,2,4-triazol,
- 5,6-Dicyano-3-methyl-pyrrolo-[2,1-c]-1,2,4-triazol,
- 7-Chlor-5,6-dicyano-3-methyl-pyrrolo-[2,1-c]-1,2,4-triazol, und
- den Additionssalzen dieser Verbindungen mit einer Säure.

16. Zusammensetzung nach einem der Ansprüche 1 bis 11, **dadurch gekennzeichnet, daß** die Verbindungen der Formel (II) ausgewählt sind unter:
a) Pyrrolo-[1,2-b]-1,2,4-triazolen der Formel: 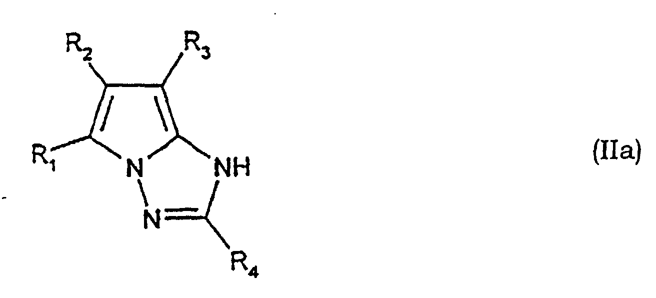
b) Pyrrolo-[2,1-c]-1,2,4-triazolen der Formel: 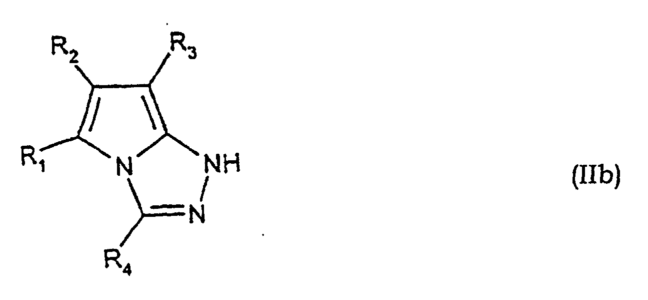
c) Pyrrolo-[1,2-c]-imidazolen der Formel: 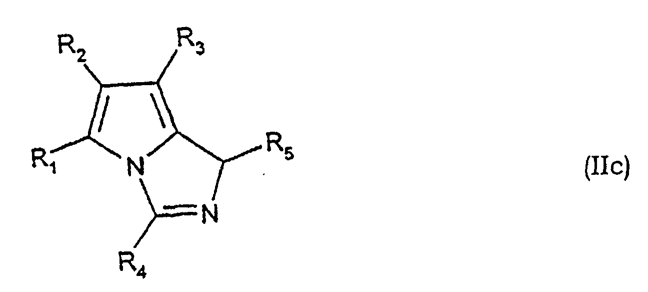
d) Pyrrolo-[1,2-e]-tetrazolen der Formel: 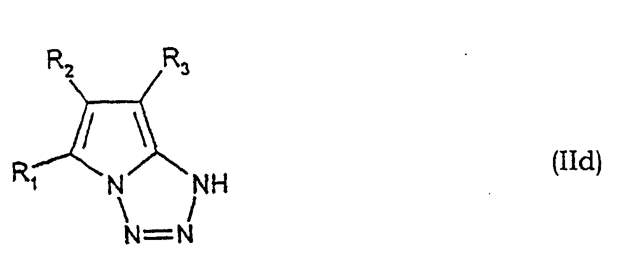
e) Pyrrolo-[1,2-a]-pyrrolen der Formel: 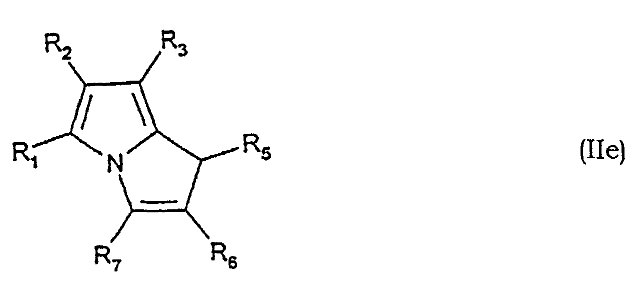
f) Pyrrolo-[1,2-a]-imidazolen der Formel: 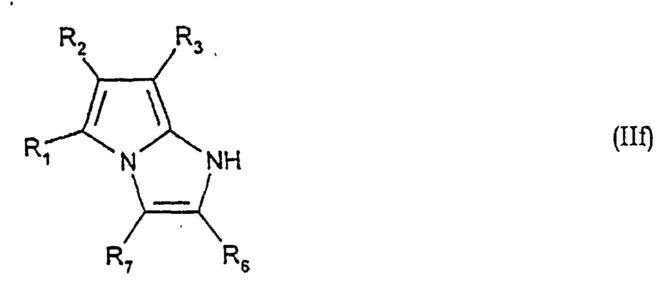
g) Pyrrolo-[1,2-c]-1,2,3-triazolen der Formel: 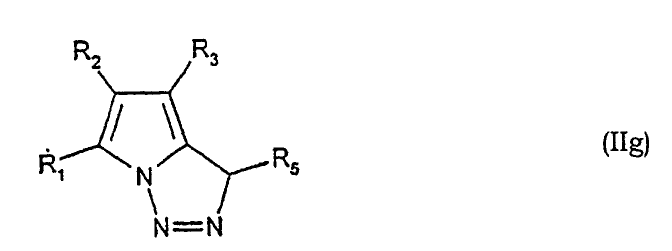
wobei die Gruppen R₁, R₂, R₃, R₄, R₅, R₆ und R₇ die in den Ansprüchen 1 bis 10 angegebenen Bedeutungen aufweisen.

17. Zusammensetzung nach Anspruch 16, **dadurch gekennzeichnet, daß** die Verbindungen der Formel (IIa) Verbindungen sind, worin bedeuten:
- R₁ Wasserstoff oder Chlor;
- R₂ bzw. R₃ Methoxycarbonyl und Cyano; Ethyloxycarbonyl und Cyano; Cyano und Methoxycarbonyl oder Ethoxycarbonyl; Cyano und Trifluormethyl; Cyano und Phenylsulfonyl; Methyloxycarbonyl und Methyloxycarbonyl; Wasserstoff und Wasserstoff; Wasserstoff und Methyl; Trifluormethyl und Cyano oder Trifluormethyl und Methyloxycarbonyl; Carboxy und Cyano; Cyano und Cyano; Ethyloxycarbonyl und Ethyloxycarbonyl; Phenyl und Cyano; und Methyl und Wasserstoff;
- R₄ Methyl, Ethyl, Isopropyl, Phenyl oder Wasserstoff.

18. Zusammensetzung nach Anspruch 16, **dadurch gekennzeichnet, daß** die Verbindungen der Formel (IIb) Verbindungen sind, worin bedeuten:
- R₁ Wasserstoff oder Chlor;
- R₂ bzw. R₃ Cyano und Methoxycarbonyl; Methoxycarbonyl und Cyano; Methoxycarbonyl und Methoxycarbonyl; Wasserstoff und Wasserstoff oder Wasserstoff und Methyl; Cyano und Cyano; Ethyloxycarbonyl und Ethyloxycarbonyl; Phenyl und Cyano; und t-Butyl und Cyano.
- R₄ Methyl, Ethyl, Isopropyl, Phenyl oder Wasserstoff.

19. Zusammensetzung nach Anspruch 16, **dadurch gekennzeichnet, daß** die Verbindungen der Formel (IIc) Verbindungen sind, worin bedeuten:
- R₁ Acetamido, Wasserstoff oder Chlor;
- R₂ bzw. R₃ Methoxycarbonyl und Cyano; Cyano und Cyano;
- R₄ Wasserstoff;
- R₅ Cyano.

20. Zusammensetzung nach Anspruch 16, **dadurch gekennzeichnet, daß** die Verbindungen der Formel (IId) Verbindungen sind, worin bedeuten:
- R₁ Wasserstoff oder Chlor;
- R₂ bzw. R₃ Cyano und Methoxycarbonyl; Cyano und Cyano; Methoxycarbonyl und Cyano; Methoxycarbonyl und Methoxycarbonyl; Wasserstoff und Wasserstoff; Wasserstoff und Methyl.

21. Zusammensetzung nach Anspruch 16, **dadurch gekennzeichnet, daß** die Verbindungen der Formel (IIe) Verbindungen sind, worin bedeuten:
- R₁ Wasserstoff oder Chlor;
- R₂ bzw. R₃ Cyano und Methoxycarbonyl;
- R₅ Trifluormethyl;
- R₆ Phenyl oder Methyl;
- R₇ Methyl.

22. Zusammensetzung nach Anspruch 16, **dadurch gekennzeichnet, daß** die Verbindungen der Formel (IIf) Verbindungen sind, worin bedeuten:
- R₁ Wasserstoff oder Chlor;
- R₂, R₃, R₆ bzw. R₇:
Methoxycarbonyl/Cyano/Cyano/Phenyl;
Cyano/Methoxycarbonyl/Cyano/Phenyl;
Cyano /Methoxycarbonyl/Methoxycarbonyl/Phenyl;
Wasserstoff/Wasserstoff/Wasserstoff/Wasserstoff;
Wasserstoff/Wasserstoff/Methyl/Methyl.

23. Zusammensetzung nach Anspruch 16, **dadurch gekennzeichnet, daß** die Verbindungen der Formel (IIg) Verbindungen sind, worin bedeuten:
- R₁ Wasserstoff oder Chlor;
- R₂ Cyano;
- R₃ Methoxycarbonyl; Ethoxycarbonyl;
- Rs Cyano.

24. Zusammensetzung nach Anspruch 16, **dadurch gekennzeichnet, daß** die Verbindungen der Formel (II) ausgewählt sind unter:
- 5-Cyano-4-Ethoxycarbonyl-8-methyl-pyrrolo-[1,2-b]-1,2,4-triazol,
- 5-Cyano-4-carboxy-8-methyl-pyrrolo-[1,2-b]-1,2,4-triazol,
- 4,5-Dicyano-8-methyl-pyrrolo-[1,2-b]-1,2,4-triazol,
- 5-Cyano-8-methyl-4-phenyl-pyrrolo-[1,2-b]-1,2,4-triazol,
- 4,8-Dimethyl-pyrrolo-[1,2-b]-1,2,4-triazol,
- 4,5-Di(ethyloxycarbonyl)-8-methyl-pyrrolo-[1,2-b]-1,2,4-triazol,
- 3-Chlor-5-cyano-4-ethoxycarbonyl-8-methyl-pyrrolo-[1,2-b]-1,2,4-triazol,
- 5-Cyano-4-Ethoxycarbonyl-8-phenyl-pyrrolo-[1,2-b]-1,2,4-triazol,
- 5-Cyano-4-carboxy-8-phenyl-pyrrolo-[1,2-b]-1,2,4-triazol,
- 4,5-Dicyano-8-phenyl-pyrrolo-[1,2-b]-1,2,4-triazol,
- 4,5-Di(ethyloxycarbonyl)-8-phenyl-pyrrolo-[1,2-b]-1,2,4-triazol,
- 3-Chlor-5-cyano-4-ethoxycarbonyl-8-phenyl-pyrrolo-[1,2-b]-1,2,4-triazol,
- 4-Cyano-5-carboxy-8-(2-nitro-5-hydroxyphenyl)-pyrrolo-[1,2-b]-1,2,4-triazol,
- 6,7-Dicyano-3-methyl-pyrrolo-[2,1-c]-1,2,4-triazol,
- 5-Chlor-6,7-dicyano-3-methyl-pyrrolo-[2,1-c]-1,2,4-triazol,
- 6,7-Di(ethyloxycarbonyl)-3-methyl-pyrrolo-[2,1-c]-1,2,4-triazol,
- 7-Cyano-3-methyl-6-phenyl-pyrrolo-[2,1-c]-1,2,4-triazol,
- 7-Cyano-3-methyl-6-t-butyl-pyrrolo-[2,1-c]-1,2,4-triazol,
- 6,8-Dicyano-5-ethoxycarbonyl-pyrrolo-[1,2-c]-imidazol,
- 4-Chlor-6,8-dicyano-5-ethoxycarbonyl-pyrrolo-[1,2-c]-imidazol,
- 6,7-Dicyano-pyrrolo-[1,2-e]-tetrazol,
- 6-Cyano-7-ethoxycarbonyl-pyrrolo-[1,2-e]-tetrazol,
- 5-Chlor-6,7-dicyano-pyrrolo-[1,2-e]-tetrazol,
- 2,3,7-Tricyano-6-methyl-pyrrolo-[1,2-a]-imidazol,
- 2,3,7-Tricyano-6-trifluormethyl-pyrrolo-[1,2-a]-imidazol,
- 2,3,7-Tricyano-6-t-butyl-pyrrolo-[1,2-a]-imidazol,
- 2,3,7-Tricyano-6-phenyl-pyrrolo-[1,2-a]-imidazol,
- 2,3,7-Tricyano-6-ethoxycarbonyl-pyrrolo-[1,2-a]-imidazol,
- 5-Chlor-2,3,7-tricyano-6-t-butyl-pyrrolo-[1,2-a]-imidazol,
- 5-Chlor-2,3,7-tricyano-6-phenyl-pyrrolo-[1,2-a]-imidazol,
- 7-Cyano-6-ethoxycarbonyl-pyrrolo-[1,2-a]-benzimidazol,
- 7-Cyano-6-phenyl-pyrrolo-[1,2-a]-benzimidazol,
- 7-Amido-6-ethoxycarbonyl-pyrrolo-[1,2-a]-benzimidazol,
- 5,6,8-Tricyano-pyrrolo-[1,2-c]-1,2,3-triazol,
- 5,8-Dicyano-6-ethoxycarbonyl-pyrrolo-[1,2-c]-1,2,3-triazol,
- 4-Chlor-5,8-dicyano-6-ethoxycarbonyl-pyrrolo-[1,2-c]-1,2,3-triazol, und
- den Additionssalzen dieser Verbindungen mit einer Säure.

25. Zusammensetzung nach einem der vorhergehenden Ansprüche, **dadurch gekennzeichnet, daß** die Additionssalze der Verbindungen der Formeln (I) oder (II) mit einer Säure unter den Hydrochloriden, Hydrobromiden, Tartraten, Tosylaten, Benzolsulfonaten, Sulfaten, Lactaten und Acetaten ausgewählt sind.

26. Zusammensetzung nach einem der vorhergehenden Ansprüche, **dadurch gekennzeichnet, daß** die Verbindung(en) der Formeln (I) und (II) 0,0005 bis 12 Gew.-% des Gesamtgewichts der Farbmittelzusammensetzung ausmachen.

27. Zusammensetzung nach Anspruch 26, **dadurch gekennzeichnet, daß** die Verbindung(en) der Formeln (I) und (II) 0,005 bis 6 Gew.-% des Gesamtgewichts der Farbmittelzusammensetzung ausmachen.

28. Zusammensetzung nach einem der vorhergehenden Ansprüche, **dadurch gekennzeichnet, daß** die Oxidationsbase(n) unter den p-Phenylendiaminen, Bisphenylalkylendiaminen, p-Aminophenolen, o-Aminophenolen, heterocyclischen Basen und deren Additionssalzen mit einer Säure ausgewählt sind.

29. Zusammensetzung nach einem der vorhergehenden Ansprüche, **dadurch gekennzeichnet, daß** die Oxidationsbase(n) etwa 0,0005 bis 12 Gew.-% des Gesamtgewichts der Farbmittelzusammensetzung ausmachen.

30. Zusammensetzung nach Anspruch 29, **dadurch gekennzeichnet, daß** die Oxidationsbase(n) etwa 0,005 bis 6 Gew.-% des Gesamtgewichts der Farbmittelzusammensetzung ausmachen.

31. Zusammensetzung nach einem der vorhergehenden Ansprüche, **dadurch gekennzeichnet, daß** sie ferner einen oder mehrere zusätzliche Kuppler, die von den Verbindungen der Formeln (I) und (II) verschieden sind, und/oder einen oder mehrere Direktfarbstoffe enthält.

32. Zusammensetzung nach einem der vorhergehenden Ansprüche, **dadurch gekennzeichnet, daß** das zum Färben geeignete Medium (oder der Träger) aus Wasser oder einem Gemisch von Wasser und mindestens einem organischen Lösungsmittel besteht, das unter den niederen C₁₋₄-Alkoholen, Glycerin, Glykolen und Glykolethern, aromatischen Alkoholen, analogen Produkten und deren Gemischen ausgewählt ist.

33. Zusammensetzung nach einem der vorhergehenden Ansprüche, **dadurch gekennzeichnet, daß** sie einen pH-Wert im Bereich von 3 bis 12 aufweist.

34. Zusammensetzung nach einem der vorhergehenden Ansprüche, **dadurch gekennzeichnet, daß** sie als Flüssigkeit, Creme oder Gel oder in beliebigen weiteren Formen, die zur Durchführung einer Färbung von Keratinfasern und insbesondere von menschlichem Haar geeignet sind, vorliegt.

35. Verwendung von Verbindungen der Formeln (I) oder (II) oder von Additionssalzen dieser Verbindungen mit einer Säure nach einem der Ansprüche 1 bis 25 als Kuppler in Zusammensetzungen zum oxidativen Färben von Keratinfasern und insbesondere menschlichen Keratinfasern, wie dem Haar, in Kombination mit mindestens einer Oxidationsbase.

36. Verfahren zum oxidativen Färben von Keratinfasern und insbesondere menschlichen Keratinfasern, wie dem Haar, **dadurch gekennzeichnet, daß** auf die Fasern mindestens eine Farbmittelzusammensetzung nach einem der Ansprüche 1 bis 34 aufgetragen wird und die Farbe bei einem sauren, neutralen oder alkalischen pH-Wert mit einem Oxidationsmittel entwickelt wird, das bei der Anwendung zu der Farbmittelzusammensetzung gegeben wird oder das in einer oxidierenden Zusammensetzung vorliegt, die gleichzeitig oder getrennt davon anschließend aufgetragen wird.

37. Verfahren nach Anspruch 36, **dadurch gekennzeichnet, daß** das Oxidationsmittel unter Wasserstoffperoxid, Harnstoffperoxid, Alkalimetallbromaten und Salzen von Persäuren, wie Perboraten und Persulfaten, ausgewählt ist.

38. Vorrichtung mit mehreren Abteilungen oder "Kit" zum Färben mit mehreren Abteilungen, wobei eine erste Abteilung eine Farbmittelzusammensetzung nach einem der Ansprüche 1 bis 34 und eine zweite Abteilung eine oxidierende Zusammensetzung enthält.

## Claims

1. Composition for dyeing keratin fibres, and in particular human keratin fibres such as the hair, **characterized in that** it comprises, in a medium that is suitable for dyeing:
- as coupler, at least one pyrroloazole compound or one of the addition salts thereof with an acid corresponding to one of the following formulae: 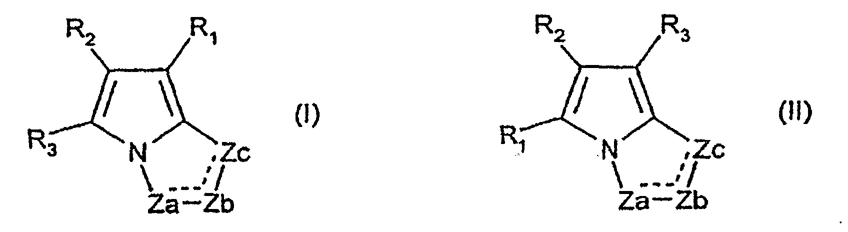 in which:
R₁ represents: a hydrogen atom; a halogen atom; an acetylamido radical; an alkoxy radical; an aryloxy radical; an acyloxy radical; an arylthio radical; an alkylthio radical; a heteroarylthio radical; a heteroaryloxy radical; a thiocyano radical; an N,N-diethylthiocarbonylthio radical; a dodecyloxythiocarbonylthio radical; a benzenesulphonamido radical; an N-ethyltoluenesulphonamido radical; a pentafluorobutanamido radical; a 2,3,4,5,6-pentafluorobenzamido radical; a p-cyanophenylureido radical; an N,N-diethylsulphamoylamino radical; a pyrazolyl radical; an imidazolyl radical; a triazolyl radical; a tetrazolyl radical; a benzimidazolyl radical; a 1-benzyl-5-ethoxy-3-hydantoinyl radical; a 1-benzyl-3-hydantoinyl radical; 5,5-dimethyl-2,4-dioxo-3-oxazolidinyl; a 2-oxy-1,2-dihydro-1-pyridyl radical; an alkylamido; an arylamido; acetylamido; a radical NR^{III}R^{IV} with R^{III} and R^{IV}, which may be identical or different, representing a C₁-C₄ alkyl, a hydroxyalkyl; a carboxyl; an alkoxycarboxylic radical;
R₂ and R₃, independently of each other, represent a hydrogen atom; a halogen atom; a linear or branched C₁-C₅ alkyl radical, optionally substituted with one or two halogen, hydroxyl, alkoxy, aryloxy, amino, alkylamino, acyl or acylamino radicals; a C₁-C₄ alkoxy radical; a C₁-C₄ alkylthio radical; an arylthio radical; a benzylthio radical; an acyl radical; an acylamino radical; an acyloxy radical; a carbamoyl radical; a phenyl radical optionally substituted with one or two halogen, nitro, sulphonyl, C₁-C₄ alkoxy, C₁-C₄ alkyl, C₁-C₃ trifluoroalkyl, amino or alkylamino radicals; an alkoxycarbonyl radical; an aryloxycarbonyl radical; a cyano radical; a nitro radical; a dialkylphosphono radical; a diarylphosphono radical; a dialkoxyphospholyl radical; a dialkylphosphinyl radical; a diarylphosphinyl radical; an alkylsulphinyl radical; an arylsulphinyl radical; an arylsulphonyl radical; an alkylsulphonyl radical; a sulphonyloxy radical; an acylthio radical; a sulphamoyl radical; a thiocyanate radical; a thiocarbonyl radical;
Zₐ, Z_{b} and Z_{c} represent, independently of each other, a nitrogen atom or a carbon atom bearing a radical R₄, R₅, R₆ or R₇;
R₄, R₆ and R₇, which may be identical or different, represent a hydrogen atom; a linear or branched C₁-C₂₀ alkyl radical, optionally substituted with 1 or 2 radicals R chosen from the group consisting of halogen, nitro, cyano, hydroxyl, alkoxy, aryloxy, amino, alkylamino, acylamino, carbamoyl, sulphonamido, sulphamoyl, imido, alkylthio, arylthio, aryl, alkoxycarbonyl and acyl; an aryl radical optionally substituted with 1 or 2 radicals R as defined above; a 5- or 6-membered heterocycle containing at least one nitrogen, oxygen or sulphur atom optionally substituted with 1 or 2 radicals R as defined above;
when R₄, R₆ and R₇ denote an alkyl radical, an aryl radical or a 5- or 6-membered heterocycle (defined above), it may be linked to the carbon atom of the nucleus via an oxygen, nitrogen or sulphur atom (in this case, R₄, R₆ and R₇ become XR₄, XR₆ or XR₇ with X = O, NH or S);
R₄, R₆ and R₇ can also denote a halogen atom; an acyl radical; a sulphonyl radical; a sulphinyl radical; a phosphonyl radical; a carbamoyl radical; a sulphamoyl radical; a cyano radical; a siloxy radical; an amino radical; an acylamino radical; an acyloxy radical; a carbamoyloxy radical; a sulphonamide radical; an imide radical; a ureido radical; a sulphamoylamino radical; an alkoxycarbonylamino radical; an aryloxycarbonylamino radical; an alkoxycarbonyl radical; an aryloxycarbonyl radical; a carboxyl radical;
R₅ denotes hydrogen; halogen; an acyl radical; an acyloxy radical; a carbamoyl radical; an alkoxycarbonyl radical; a cyano radical; a nitro radical; a dialkylphosphono radical; a diarylphosphono radical; a dialkoxyphospholyl radical; a dialkylphosphinyl radical; a diarylphosphinyl radical; an alkylsulphinyl radical; an arylsulphinyl radical; an alkylsulphonyl radical; an arylsulphonyl radical; a sulphonyloxy radical; an acylthio radical; a sulphamoyl radical; a thiocyanate radical; a thiocarbonyl radical; a halogenated aryloxy radical; a halogenated alkylamino radical; a halogenated alkylthio; an aryl optionally substituted with electron-withdrawing groups; a heterocycle;
- and at least one oxidation base.

2. Composition according to Claim 1, **characterized in that** the radicals R₁ of formulae (I) and (II) are chosen from the group consisting of:
a hydrogen atom; a C₁-C₄ alkoxy; phenoxy; phenoxy substituted with a halogen atom, a C₁-C₄ alkyl, a carboxyl or a trifluoromethyl group; an acyloxy radical; benzyloxy; C₁-C₄ alkylthio; phenylthio; phenylthio substituted with a halogen atom, a C₁-C₄ alkyl, a carboxyl or a trifluoromethyl group; a C₁-C₄ alkylamido; phenylamido; a radical NR^{III}R^{IV} with R^{III} and R^{IV}, which may be identical or different, representing a C₁-C₄ alkylor a C₁-C₄ hydroxyalkyl; a carboxyl; a C₁-C₄ alkoxycarboxylic radical.

3. Composition according to Claim 2, **characterized in that** the radicals R₁ for formulae (I) and (II) are chosen from the group consisting of:
hydrogen; chlorine or bromine; methoxy or ethoxy; phenyloxy; 4-methylphenyloxy; acyloxy; benzyloxy; methylthio or ethylthio; phenylthio; 4-methylphenylthio; 2-tert-butylphenylthio; acetamido; phenylacetamido; dimethylamino; diethylamino; ethylmethylamino; (β-hydroxyethyl)methylamino.

4. Composition according to Claim 2 or 3, **characterized in that** the radicals R₁ of formulae (I) and (II) are chosen from the group consisting of:
hydrogen; chlorine; ethoxy; phenoxy; benzyloxy; acyloxy; acetamido; dimethylamino.

5. Composition according to any one of Claims 1 to 4, **characterized in that** the radicals R₂ and R₃ of formulae (I) and (II) are chosen from the group consisting of:
acyl; acyloxy; carbamoyl; alkoxycarbonyl; aryloxycarbonyl; cyano; nitro; alkylsulphinyl; arylsulphinyl; alkylsulphonyl; arylsulphonyl; sulphamoyl; halogenated alkyl; C₁-C₄ alkyl, hydrogen.

6. Composition according to Claim 5, **characterized in that** the radicals R₂ and R₃ of formulae (I) and (II) are chosen from the group consisting of:
acyl; alkoxycarbonyl; nitro; cyano; arylsulphonyl; carbamoyl; halogenated alkyl; hydrogen; C₁-C₄ alkyl.

7. Composition according to Claim 5 or 6, **characterized in that** the radicals R₂ of formulae (I) and (II) are chosen from the group consisting of:
cyano; hydrogen; methyl; phenyl, and **in that** the radicals R₃ of formulae (I) and (II) are chosen from the group consisting of: alkoxycarbonyl such as methoxycarbonyl or ethoxycarbonyl; aryloxycarbonyl; hydrogen; methyl; cyano.

8. Composition according to any one of Claims 1 to 7, **characterized in that** the radicals R₄, R₆ and R₇ of formulae (I) and (II) are chosen from the group consisting of:
a linear or branched C₁-C₄ alkyl radical; aryl; phenyl substituted with a halogen, a methoxy radical, a nitro group, a cyano group, a trifluoromethyl group or an amino group; cyano; nitro; acylamino; arylamino; alkylthio; arylthio; carbamoyl; sulphonyl; alkoxycarbonyl; aryloxycarbonyl; acyl, hydrogen.

9. Composition according to Claim 8, **characterized in that** the radicals R₄, R₆ and R₇ of formulae (I) and (II) are chosen from the group consisting of:
hydrogen; linear or branched C₁-C₄ alkyl; aryl; phenyl substituted with a halogen, a methoxy radical, a nitro group, a cyano group, a trifluoromethyl group or an amino group.

10. Composition according to any one of Claims 1 to 9, **characterized in that** the radicals R₅ of formulae (I) and (II) are chosen from the group consisting of:
acyl; alkoxycarbonyl; aryloxycarbonyl; nitro; cyano; arylsulphonyl; halogenated alkyl; hydrogen.

11. Composition according to any one of Claims 1 to 10, **characterized in that** the radicals R₅ are chosen from the group consisting of: cyano; alkoxycarbonyl; aryloxycarbonyl; halogenated alkyl; a hydrogen.

12. Composition according to any one of Claims 1 to 11, **characterized in that** the compounds of formula (I) are chosen from the group consisting of:
(i) the pyrrolo[1,2-b]-1,2,4-triazoles of formula: 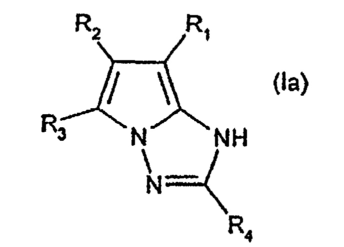
(ii) the pyrrolo[2,1-c]-1,2,4-triazoles of formula: 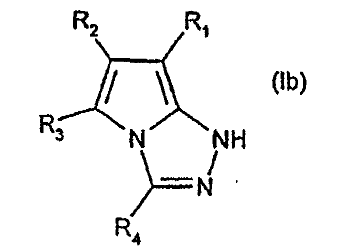
in which R₁, R₂, R₃ and R₄ have the same meanings as those given in any one of Claims 1 to 11.

13. Composition according to Claim 12, **characterized in that** the compounds of formula (Ia) are those for which:
- R₁ denotes hydrogen or chlorine;
- R₂ and R₃ denote, respectively: cyano and cyano; ethoxycarbonyl and cyano; trifluoromethyl and cyano; phenylsulphonyl and cyano; trifluoromethyl and ethyloxycarbonyl; ethoxycarbonyl and ethoxycarbonyl; methoxycarbonyl and methoxycarbonyl; hydrogen and hydrogen or hydrogen and methyl;
- R₄ denotes methyl, ethyl, isopropyl, phenyl or hydrogen.

14. Composition according to Claim 12, **characterized in that** the compounds of formula (Ib) are those for which:
- R₁ denotes hydrogen or chlorine;
- R₂ and R₃ simultaneously denote: cyano or hydrogen;
- R₄ denotes methyl, ethyl, isopropyl, phenyl or hydrogen.

15. Composition according to Claim 12, **characterized in that** the compounds of formula (I) are chosen from:
- 3,4-dicyano-8-methylpyrrolo[1,2-b]-1,2,4-triazole,
- 3,4-dicyano-8-phenylpyrrolo[1,2-b]-1,2,4-triazole,
- 3,4-dicyano-8-tert-butylpyrrolo[1,2-b]-1,2,4-triazole,
- 5-chloro-3,4-dicyano-8-methylpyrrolo[1,2-b]-1,2,4-triazole,
- 5,6-dicyano-3-methylpyrrolo[2,1-c]-1,2,4-triazole,
- 7-chloro-5,6-dicyano-3-methylpyrrolo[2,1-c]-1,2,4-triazole,
and the addition salts thereof with an acid.

16. Composition according to any one of Claims 1 to 11, **characterized in that** the compounds of formula (II) are chosen from the group consisting of:
a) the pyrrolo[1,2-b]-1,2,4-triazoles of formula: 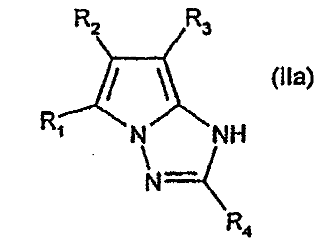
b) the pyrrolo[2,1-c]-1,2,4-triazoles of formula: 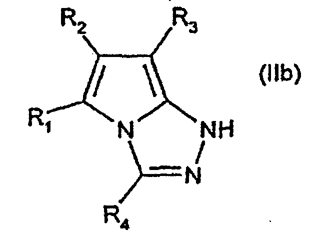
c) the pyrrolo[1,2-c]imidazoles of formula: 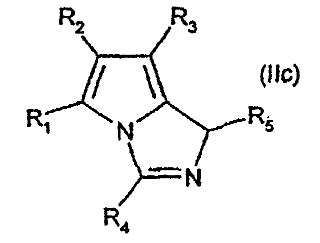
d) the pyrrolo[1,2-e]tetrazoles of formula: 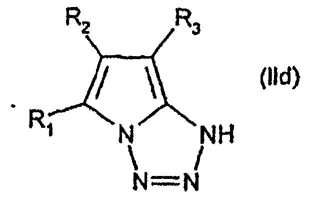
e) the pyrrolo[1,2-a]pyrroles of formula: 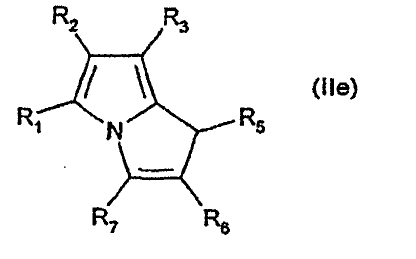
f) the pyrrolo[1,2-a]imidazoles of formula: 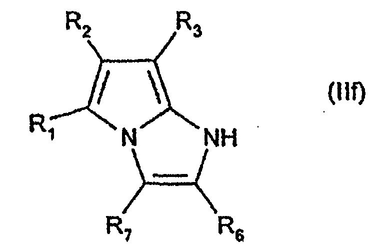
g) the pyrrolo[1,2-c]-1,2,3-triazoles of formula: 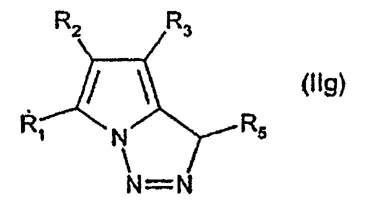
in which R₁, R₂, R₃, R₄, R₅, R₆ and R₇ have the same meanings as those given in any one of Claims 1 to 10.

17. Composition according to Claim 16, **characterized in that** the compounds of formula (IIa) are those for which:
- R₁ denotes hydrogen or chlorine;
- R₂ and R₃ denote, respectively: methoxycarbonyl and cyano; ethyloxycarbonyl and cyano; cyano and methoxycarbonyl or ethoxycarbonyl; cyano and trifluoromethyl; cyano and phenylsulphonyl; methyloxycarbonyl and methyloxycarbonyl; hydrogen and hydrogen; hydrogen and methyl; trifluoromethyl and cyano or trifluoromethyl and methyloxycarbonyl; carboxy and cyano; cyano and cyano; ethyloxycarbonyl and ethyloxycarbonyl; phenyl and cyano; methyl and hydrogen;
- R₄ denotes methyl, ethyl, isopropyl, phenyl or hydrogen.

18. Composition according to Claim 16, **characterized in that** the compounds of formula (IIb) are those for which:
- R₁ denotes hydrogen or chlorine;
- R₂ and R₃ denote, respectively: cyano and methoxycarbonyl; methoxycarbonyl and cyano; methoxycarbonyl and methoxycarbonyl; hydrogen and hydrogen or hydrogen and methyl; cyano and cyano; ethyloxycarbonyl and ethyloxycarbonyl; phenyl and cyano; tert-butyl and cyano;
- R₄ denotes methyl, ethyl, isopropyl, phenyl or hydrogen.

19. Composition according to Claim 16, **characterized in that** the compounds of formula (IIc) are those for which:
- R₁ denotes acetamido, hydrogen or chlorine;
- R₂ and R₃ denote, respectively: methoxycarbonyl and cyano; cyano and cyano;
- R₄ denotes hydrogen;
- R₅ denotes cyano.

20. Composition according to Claim 16, **characterized in that** the compounds of formula (IId) are those for which:
- R₁ denotes hydrogen or chlorine;
- R₂ and R₃ denote, respectively: cyano and methoxycarbonyl; cyano and cyano; methoxycarbonyl and cyano; methoxycarbonyl and methoxycarbonyl; hydrogen and hydrogen; hydrogen and methyl.

21. Composition according to Claim 16, **characterized in that** the compounds of formula (IIe) are those for which:
- R₁ denotes hydrogen or chlorine;
- R₂ and R₃ denote, respectively: cyano and methoxycarbonyl;
- R₅ denotes trifluoromethyl;
- R₆ denotes phenyl or methyl;
- R₇ denotes methyl.

22. Composition according to Claim 16, **characterized in that** the compounds of formula (IIf) are those for which:
- R₁ denotes hydrogen or chlorine;
- R₂, R₃, R₆ and R₇ denote, respectively:
methoxycarbonyl/cyano/cyano/phenyl;
cyano/methoxycarbonyl/cyano/phenyl;
cyano/methoxycarbonyl/methoxycarbonyl/phenyl;
hydrogen/hydrogen/hydrogen/hydrogen;
hydrogen/hydrogen/methyl/methyl.

23. Composition according to Claim 16, **characterized in that** the compounds of formula (IIg) are those for which:
- R₁ denotes hydrogen or chlorine;
- R₂ denotes cyano;
- R₃ denotes methoxycarbonyl; ethyloxycarbonyl;
- R₅ denotes cyano.

24. Composition according to Claim 16, **characterized in that** the compounds of formula (II) are chosen from:
- 5-cyano-4-ethoxycarbonyl-8-methylpyrrolo[1,2-b]-1,2,4-triazole,
- 5-cyano-4-carboxyl-8-methylpyrrolo[1,2-b]-1,2,4-triazole,
- 4,5-dicyano-8-methylpyrrolo[1,2-b]-1,2,4-triazole,
- 5-cyano-8-methyl-4-phenylpyrrolo[1,2-b]-1,2,4-triazole,
- 4,8-dimethylpyrrolo[1,2-b]-1,2,4-triazole,
- 4,5-di(ethyloxycarbonyl)-8-methylpyrrolo[1,2-b]-1,2,4-triazole,
- 3-chloro-5-cyano-4-ethoxycarbonyl-8-methylpyrrolo-[1,2-b]-1,2,4-triazole,
- 5-cyano-4-ethoxycarbonyl-8-phenylpyrrolo[1,2-b]-1,2,4-triazole,
- 5-cyano-4-carboxy-8-phenylpyrrolo[1,2-b]-1,2,4-triazole,
- 4,5-dicyano-8-phenylpyrrolo[1,2-b]-1,2,4-triazole,
- 4,5-di(ethyloxycarbonyl)-8-phenylpyrrolo[1,2-b]-1,2,4-triazole,
- 3-chloro-5-cyano-4-ethoxycarbonyl-8-phenylpyrrolo-[1,2-b]-1,2,4-triazole,
- 4-cyano-5-carboxy-8-(2-nitro-5-hydroxyphenyl)pyrrolo-[1,2-b]-1,2,4-triazole,
- 6,7-dicyano-3-methylpyrrolo[2,1-c]-1,2,4-triazole,
- 5-chloro-6,7-dicyano-3-methylpyrrolo[2,1-c]-1,2,4-triazole,
- 6,7-di(ethyloxycarbonyl)-3-methylpyrrolo[2,1-c]-1,2,4-triazole,
- 7-cyano-3-methyl-6-phenylpyrrolo[2,1-c]-1,2,4-triazole,
- 7-cyano-3-methyl-6-tert-butylpyrrolo[2,1-c]-1,2,4-triazole,
- 6,8-dicyano-5-ethoxycarbonylpyrrolo[1,2-c]imidazole,
- 4-chloro-6,8-dicyano-5-ethoxycarbonylpyrrolo[1,2-c]-imidazole,
- 6,7-dicyanopyrrolo[1,2-e]tetrazole,
- 6-cyano-7-ethoxycarbonylpyrrolo[1,2-e]tetrazole,
- 5-chloro-6,7-dicyanopyrrolo[1,2-e]tetrazole,
- 2,3,7-tricyano-6-methylpyrrolo[1,2-a]imidazole,
- 2,3,7-tricyano-6-trifluoromethylpyrrolo[1,2-a]-imidazole,
- 2,3,7-tricyano-6-tert-butylpyrrolo[1,2-a]imidazole,
- 2,3,7-tricyano-6-phenylpyrrolo[1,2-a]imidazole,
- 2,3,7-tricyano-6-ethoxycarbonylpyrrolo[1,2-a]-imidazole,
- 5-chloro-2,3,7-tricyano-6-tert-butylpyrrolo[1,2-a]-imidazole,
- 5-chloro-2,3,7-tricyano-6-phenylpyrrolo[1,2-a]-imidazole,
- 7-cyano-6-ethoxycarbonylpyrrolo[1,2-a]benzimidazole,
- 7-cyano-6-phenylpyrrolo[1,2-a]benzimidazole,
- 5,6,8-tricyanopyrrolo[1,2-c]-1,2,3-triazole,
- 7-amido-6-ethoxycarbonylpyrrolo[1,2-a]benzimidazole,
- 5,8-dicyano-6-ethoxycarbonylpyrrolo[1,2-c]-1,2,3-triazole,
- 4-chloro-5,8-dicyano-6-ethoxycarbonylpyrrolo[1,2-c]-1,2,3-triazole,
and the addition salts thereof with an acid.

25. Composition according to any one of the preceding claims, **characterized in that** the addition salts with an acid of the compounds of formula (I) or (II) are chosen from the hydrochlorides, hydrobromides, tartrates, tosylates, benzenesulphonates, sulphates, lactates and acetates.

26. Composition according to any one of the preceding claims, **characterized in that** the compound(s) of formula (I) or (II) represent(s) from 0.0005% to 12% by weight relative to the total weight of the dye composition.

27. Composition according to Claim 26, **characterized in that** the compound(s) of formula (I) or (II) represent(s) from 0.005% to 6% by weight relative to the total weight of the dye composition.

28. Composition according to any one of the preceding claims, **characterized in that** the oxidation base(s) is (are) chosen from para-phenylenediamines, bis(phenyl)alkylenediamines, para-aminophenols, ortho-aminophenols and heterocyclic bases, and the addition salts thereof with an acid.

29. Composition according to any one of the preceding claims, **characterized in that** the oxidation base(s) represent(s) from 0.0005% to 12% by weight approximately relative to the total weight of the dye composition.

30. Composition according to Claim 29, **characterized in that** the oxidation base(s) represent(s) from 0.005% to 6% by weight approximately relative to the total weight of the dye composition.

31. Composition according to any one of the preceding claims, **characterized in that** it also contains one or more additional couplers other than the compounds of formula (I) or (II) and/or one or more direct dyes.

32. Composition according to any one of the preceding claims, **characterized in that** the medium that is suitable for dyeing (or the support) consists of water or of a mixture of water and at least one organic solvent chosen from C₁-C₄ lower alkanols, glycerol, glycols and glycol ethers, aromatic alcohols, similar products and mixtures thereof.

33. Composition according to any one of the preceding claims, **characterized in that** it has a pH of between 3 and 12.

34. Composition according to any one of the preceding claims, **characterized in that** it is in the form of a liquid, a cream, a gel or any other form that is suitable for dyeing keratin fibres, and especially human hair.

35. Use of the compounds of formula (I) or (II) or of the addition salts thereof with an acid as defined in any one of Claims 1 to 25, as couplers in compositions for the oxidation dyeing of keratin fibres, and in particular of human keratin fibres such as the hair, in combination with at least one oxidation base.

36. Process for the oxidation dyeing of keratin fibres, and in particular of human keratin fibres such as the hair, **characterized in that** at least one dye composition as defined in any one of Claims 1 to 34 is applied to these fibres, the colour being developed at acidic, neutral or alkaline pH with the aid of an oxidizing agent that is added just at the time of use to the dye composition, or that is present in an oxidizing composition applied simultaneously or sequentially in a separate manner.

37. Process according to Claim 36, **characterized in that** the oxidizing agent is chosen from hydrogen peroxide, urea peroxide, alkali metal bromates, and persalts such as perborates and persulphates.

38. Multi-compartment device or multi-compartment dyeing "kit", a first compartment of which contains a dye composition as defined in any one of Claims 1 to 34, and a second compartment of which contains an oxidizing composition.
